# EUROPEAN PATENT SPECIFICATION

(11) **EP 3 697 386 B1**
(45) Date of publication and mention of the grant of the patent: **26.07.2023**
(21) Application number: 18868801.4
(22) Date of filing: 19.10.2018
(51) Int. Cl.: A61K 9/127, A61K 9/00, A61K 31/506, A61K 31/683, A61K 31/685, A61K 31/445, A61K 31/12, A61K 31/4545, A61P 9/00, A61P 9/02, A61P 9/04, A61P 9/06, A61P 35/00, A61P 37/08

(54) **LIQUID COMPOSITIONS FOR PERORAL USE COMPRISING PHOSPHATIDYLGLYCEROL DERIVATIVES AND THIXOTROPIC EXCIPIENTS FOR TREATING CARDIAC CHANNELOPATHIES CAUSED BY PHARMACOLOGICAL AGENTS**
FLÜSSIGE ZUSAMMENSETZUNGEN ZUR PERORALEN VERWENDUNG UMFASSEND PHOSPHATIDYLGLYCEROL DERIVATE UND THIXOTROPISCHE HILFSSTOFFE ZUR BEHANDLUNG VON HERZKANALOPATHIEN VERURSACHT DURCH PHARMAKOLOGISCHE WIRKSTOFFE
COMPOSITIONS LIQUIDES POUR L'USAGE PÉRORAL COMPRENNANT DÉRIVÉS DE PHOSPHATIDYLGLYCEROL ET EXCIPIENTS THIXOTROPIQUES POUR LE TRAITEMENT DE CANALOPATIES CARDIAQUES CAUSEÉS PAR AGENTS PHARMACOLOGIQUES

(30) Priority: 19.10.2017 US 201715788683
(43) Date of publication of application: 26.08.2020
(60) Divisional application: 23175714.7
(73) Proprietor: Signpath Pharma, Inc., Sandy, UT 84092 (US)
(72) Inventor: HELSON, Lawrence, Quakertown PA 18951 (US); SHOPP, George, M., Boulder CO 80302 (US); BOUCHARD, Annie, Stoke QC JOB 3GO (CA); MAJEED, Muhammed, East Windsor NJ 08520 (US)
(74) Representative: SONN Patentanwälte OG
(86) International application number: PCT/US2018/056721
(87) International publication number: WO 2019/079726

(56) References cited:
- WO-A2-2012/167212
- US-A1- 2005 233 970
- US-A1- 2014 065 061
- US-A1- 2015 343 063
- US-A1- 2017 035 887
- SHOPP, G. M. et al.: "Liposomes ameliorate Crizotinib- and Nilotinib- induced inhibition of the cardiac IKr channel and QTc prolongation", Anticancer Research, vol. 34, 2014, pages 4733-4740, XP055480638,

## Description

### TECHNICAL FIELD OF THE INVENTION

The present invention relates in general to the field of drug treatment, and more particularly, to novel compositions and methods for reducing or eliminating channelopathies or conditions resulting from irregularities or alterations in cardiac patterns caused by an active agent or a drug, and one or more organoleptic, thixotropic, or both organoleptic and thixotropic agents.

### BACKGROUND OF THE INVENTION

Without limiting the scope of the invention, its background is described in connection with compositions and methods for controlling the duration of repolarization of the cardiac ventricle QT in a subject comprising administering to subject in need thereof of a modification of or functional interference with a therapeutic agent, or congenital defect which if unmodified can induce prolongation of repolarization in the heart myocyte action potential, torsade de points, and the long QT syndrome.

The beating of the heart is due to precisely controlled regularly spaced waves of myocardial excitation and contraction. The electrical currents during ion-based depolarization and repolarization can be measured by electrical leads placed on the body in specific locations (the electrocardiogram) which measure electrical waves. The P-wave represents a wave of depolarization in the atrium. When the entire atria becomes depolarized, the wave returns to zero. After 0.1 seconds the ventricle is entirely depolarized resulting in the QRS complex. The three peaks are due to the way the current spreads in the ventricles. This is followed by the T-wave or repolarization of the ventricle. The QT interval measured from the beginning of the QRS complex to the end of the T wave on the standard ECG represents the duration till the completion of the repolarization phase of the cardiac myocyte (or the depolarization and repolarization of the ventricle). The duration of this interval can vary due to genetic variation, cardiac disease, electrolyte balance, envenomation, and drugs. Prolongation of the QT interval can result in ventricular arrhythmias and sudden death.

Drug induced long QTc Syndrome (LQTS) i.e., a prolongation of the action potential duration is a common cause of governmental mandated drug withdrawal. QTc prolongation is an unpredictable risk factor for *Torsades de Pointes* (TdP), a polymorphic ventricular tachycardia leading to ventricular fibrillation. Drug induced LQTS comprises about 3% of all prescriptions which when followed by TdP may constitute a lethal adverse reaction. Patients taking one or more than one QTc-prolonging drug concomitantly, have an enhanced risk of TdP. While the overall occurrence of TdP is statistically rare but clinically significant for the affected individual, assay for this drug effect is a mandatory requirement prior to allowing a drug to enter clinical trials.

Common structurally diverse drugs block the human ether-a-go-go-related gene (KCNH2 or hERG) coded K⁺ channel and the cardiac delayed-rectifier potassium current I_{K} (KV11.1) resulting in acquired LQTS. Drug-associated increased risk of LQTS is a major drug development hurdle and many drugs have been withdrawn during pre-clinical development, or assigned black box warnings following approval or withdrawn from the market. Autosomal recessive or dominant LQTS based upon 500 possible mutations in 10 different genes coding for the potassium channel has an incidence of 1:3000 or about 100 ,000 persons in the US. Prolonged QT intervals, or risk of LQTS occur in 2.5% of the asymptomatic US population. This syndrome when expressed can lead to severe cardiac arrhythmia and sudden death in untreated patients. The probability of cardiac death in patients with asymptomatic congenital LQTS who are medicated with LQTS-inducing drugs is increased.

The majority of the acquired LTQS drug withdrawals are due to obstruction of the potassium ion channels coded by the human ether-a-go-go related gene (hERG). High concentrations of hERG blocking drugs generally induce a prolonged QTc interval and increase the probability of TdP. Up to 10% of cases of drug-induced TdP can be due to due to 13 major genetic mutations, 471 different mutations, and 124 polymorphisms (Chig, C 2006).

Systems and methods for detection of LQTS have been described previously. For example U.S. Patent Publication No. 2010/0004549 (Kohls et al. 2010) discloses a system and method of detecting LQTS in a patient by comparing a collected set of ECG data from the patient to a plurality of databases of collected ECG data. The plurality of databases will include a database containing previous ECGs from the patient, a known acquired LQTS characteristics database, and a known genetic LQTS characteristics database. Comparing the patient's ECG to these databases will facilitate the detection of such occurrences as changes in QT interval from success of ECGs, changes in T-wave morphology, changes in U-wave morphology, and can match known genetic patterns of LQTS. The system and method is sensitive to patient gender and ethnicity, as these factors have been shown to effect LQTS, and is furthermore capable of matching a QT duration to a database of drug effects. The system and method is also easily integrated into current ECG management systems and storage devices.

A system and method for the diagnosis and treatment of LQTS is described in U.S. Patent Publication No. 2008/0255464 (Michael, 2008). The Michael invention includes a system for diagnosing Long QT Syndrome (LQTS) derives a QT/QS2 ratio from an electrical systole (QT) and a mechanical systole (QS2) to detect a prolonged QT interval in a patient's cardiac cycle. A processor acquires the systoles from a microphone and chest electrodes, calculates the QT/QS2 ratio, and outputs the result to a display. The processor may compare the QT/QS2 ratio to a threshold value stored in memory for diagnosing LQTS in the patient. A user interface provides for programming, set-up, and customizing the display. A mode selector allows the system to operate alternatively as a phonocardiograph, a 12 lead electrocardiograph, or a machine for diagnosing LQTS. A related method for diagnosing cardiac disorders such as LQTS includes measuring QT and QS2 during a same cardiac cycle, calculating a QT/QS2 ratio, and comparing the result to a threshold value derived from empirical data. The method may include measuring systoles both at rest and during exercise, and may be used for drug efficacy, dosage optimization, and acquired LQTS causality tests.

A method for the treatment of cardiac arrhythmias is provided in U.S. Patent Publication No. 2007/0048284 (Donahue and Marban, 2007). The method includes administering an amount of at least one polynucleotide that modulates an electrical property of the heart. The polynucleotides of the invention may also be used with a microdelivery vehicle such as cationic liposomes and adenoviral vectors.

Methods, compositions, dosing regimes, and routes of administration for the treatment or prevention of arrhythmias have been described by Fedida et al. (2010) in U.S. Patent Publication No. 2001/00120890. In the Fedida invention, early after depolarizations and prolongation of QT interval may be reduced or eliminated by administering ion channel modulating compounds to a subject in need thereof. The ion channel modulating compounds may be cycloalkylamine ether compounds, particularly cyclohexylamine ether compounds. Also described are compositions of ion channel modulating compounds and drugs which induce early after depolarizations, prolongation of QT interval and/or Torsades de Pointes. The Fedida invention also discloses antioxidants which may be provided in combination with the ion channel modulating compounds, non-limiting examples of the antioxidants include vitamin C, vitamin E, beta-carotene, lutein, lycopene, vitamin B2, coenzyme Q10, cysteine as well as herbs, such as bilberry, turmeric (curcumin), grape seed or pine bark extracts, and ginkgo.

Further compositions and/or methods relating to cardiac channelopathies, QT prolongation or other fields are known from US 2015/343063 A1, Shopp George M et al. (Anticancer Research 34.9 (2014): 4733-4740), WO 2012/167212 A2, US 2014/065061 A1, US 2005/233970 A1 and US 2017/035887 A1.

### SUMMARY OF THE INVENTION

In one embodiment, the present invention includes a composition for use in preventing one or more cardiac channelopathies or conditions resulting from irregularities or alterations in cardiac patterns caused by an active agent or a drug in a human or animal subject comprising: an amount of a phosphatidylglycerol adapted for oral administration effective to reduce or prevent one or more cardiac channelopathies or conditions resulting from irregularities or alterations in cardiac patterns caused by the active agent or drug; and one or more thixotropic agent, or both organoleptic and thixotropic agents, wherein the composition is a liquid and the thixotropic agent forms a thixotrophic matrix and is selected from at least one of polysaccharides, cellulose, carboxymethylcellulose, gums, xanthan gum, collagen, gelatin, aerogels, polyacrylamide, alkyd resins, or silica-lipids; wherein the drug is selected from Albuterol (salbutamol), Alfuzosin, Amantadine, Amiodarone, Amisulpride, Amitriptyline, Amoxapine, Amphetamine, Anagrelide, Apomorphine, Arformoterol, Aripiprazole, Arsenic trioxide, Astemizole, Atazanavir, Atomoxetine, Azithromycin, Bedaquiline, Bepridil, Bortezomib, Bosutinib, Chloral hydrate, Chloroquine, Chlorpromazine, Ciprofloxacin, Cisapride, Citalopram, Clarithromycin, Clomipramine, Clozapine, Cocaine, Crizotinib, Dabrafenib, Dasatinib, Desipramine, Dexmedetomidine, Dexmethylphenidate, Dextroamphetamine (d-Amphetamine), Dihydroartemisinin+piperaquine, Diphenhydramine, Disopyramide, Dobutamine, Dofetilide, Dolasetron, Domperidone, Dopamine, Doxepin, Dronedarone, Droperidol, Ephedrine, Epinephrine (Adrenaline), Eribulin, Erythromycin, Escitalopram, Famotidine, Felbamate, Fenfluramine, Fingolimod, Flecainide, Fluconazole, Fluoxetine, Formoterol, Foscarnet, Fosphenytoin, Furosemide (Frusemide), Galantamine, Gatifloxacin, Gemifloxacin, Granisetron, Halofantrine, Haloperidol, Hydrochlorothiazide, Ibutilide, Iloperidone, Imipramine (melipramine), Indapamide, Isoproterenol, Isradipine, Itraconazole, Ivabradine, Ketoconazole, Lapatinib, Levalbuterol (levsalbutamol), Levofloxacin, Levomethadyl, Lisdexamfetamine, Lithium, Mesoridazine, Metaproterenol, Methadone, Methamphetamine (methamfetamine), Methylphenidate, Midodrine, Mifepristone, Mirabegron, Mirtazapine, Moexipril/HCTZ, Moxifloxacin, Nelfinavir, Nicardipine, Nilotinib, Norepinephrine (noradrenaline), Norfloxacin, Nortriptyline, Ofloxacin, Olanzapine, Ondansetron, Oxytocin, Paliperidone, Paroxetine, Pasireotide, Pazopanib, Pentamidine, Perflutren lipid microspheres, Phentermine, Phenylephrine, Phenylpropanolamine, Pimozide, Posaconazole, Probucol, Procainamide, Promethazine, Protriptyline, Pseudoephedrine, Quetiapine, Quinidine, Quinine sulfate, Ranolazine, Rilpivirine, Risperidone, Ritodrine, Ritonavir, Roxithromycin, Salmeterol, Saquinavir, Sertindole, Sertraline, Sevoflurane, Sibutramine, Solifenacin, Sorafenib, Sotalol, Sparfloxacin, Sulpiride, Sunitinib, Tacrolimus, Tamoxifen, Telaprevir, Telavancin, Telithromycin, Terbutaline, Terfenadine, Tetrabenazine, Thioridazine, Tizanidine, Tolterodine, Toremifene, Trazodone, Trimethoprim-Sulfa, Trimipramine, Vandetanib, Vardenafil, Vemurafenib, Venlafaxine, Voriconazole, Vorinostat, or Ziprasidone; and wherein the phosphatidylglycerol is selected from lysophosphatidylcholine, lauroyl-lysophosphatidylcholine, myristoyl-lysophosphatidylcholine, palmitoyl-lysophosphatidylcholine, stearoyl-lysophosphatidylcholine, arachidoyl-lysophosphatidylcholine, oleoyl-lysophosphatidylcholine, linoleoyl-lysophosphatidylcholine, linolenoyl-lysophosphatidylcholine or erucoyl-lysophosphatidylcholine.

In one aspect, the organoleptic agents include one or more flavorants, sweeteners, coolants, dyes, or combinations and mixtures thereof. Moreover, it has been found that in a powder dried according to the invention undesired organoleptic changes have been hardly effected, if effected at all, and that a powder dried according to the invention has sufficient solubility for various applications. The thixotropic agent forms a thixotrophic matrix, e.g., polysaccharides such as cellulose (e.g., carboxymethylcellulose) or gums (e.g., xanthan), collagen, gelatin, aerogels, polyacrylamide, alkyd resins, and silica-lipids. In one aspect, the composition includes both organoleptic and thixotropic agents. In one aspect, the phosphatidylglycerol is provided in the form of empty liposomes with a diameter of 10, 20, 25, 30, 40, 50, 60, 75, 80, 90, or 100 nM, e.g., 1-Myristoyl-2-Hydroxy-sn-Glycero-3-Phosphocholine (DMPC), 12-Mysteroyl-2-Hydroxy-sn-Glycero-3-[Phospho-rac-(glycerol)] (DMPG), or DMPC/DMPG liposomes. The lysophosphatidylglycerol includes at least one of a lysophosphatidylcholine, lauroyl-lysophosphatidylcholine, myristoyl-lysophosphatidylcholine, palmitoyl-lysophosphatidylcholine, stearoyl-lysophosphatidylcholine, arachidoyl-lysophosphatidylcholine, oleoyl-lysophosphatidylcholine, linoleoyl-lysophosphatidylcholine, linolenoyl-lysophosphatidylcholine or erucoyl-lysophosphatidylcholine; and one or more organoleptic or thixotropic agents.

A composition for use according to the invention is thus suitable for preparing a product consumable without health risks, optionally after reconstitution in a suitable liquid. In another aspect, the lysophosphatidylglycerol include at least one or 1-Myristoyl-2-Hydroxy-sn-Glycero-3-Phosphocholine (DMPC), 12-Mysteroyl-2-Hydroxy-sn-Glycero-3-[Phospho-rac-(glycerol)] (DMPG), DMPC/DMPG, 1-myristoyl-2-hydroxy-*sn*-glycero-3-phospho-(1'-*rac*-glycerol) (LysoPG), or 1-myristoyl-2-hydroxy-sn-glycero-3-phosphocholine (LysoPC). In one aspect, the organoleptic agents include one or more flavorants, sweeteners, coolants, dyes, or combinations and mixtures thereof. Moreover, it has been found that in a powder dried according to the invention undesired organoleptic changes have been hardly effected, if effected at all, and that a powder dried according to the invention has sufficient solubility for various applications. The thixotropic agent forms a thixotrophic matrix, e.g., polysaccharides such as cellulose (e.g., carboxymethylcellulose) or gums (e.g., xanthan), collagen, gelatin, aerogels, polyacrylamide, alkyd resins, and silica-lipids. In one aspect, the composition includes both organoleptic and thixotropic agents. In one aspect, the phosphatidylglycerol is provided in the form of empty liposomes with a diameter of 10, 20, 25, 30, 40, 50, 60, 75, 80, 90, or 100 nM, e.g., 1-Myristoyl-2-Hydroxy-sn-Glycero-3 -Phosphocholine (DMPC), 12-Mysteroyl-2-Hydroxy-sn-Glycero-3 - [Phospho-rac-(glycerol)] (DMPG), or DMPC/DMPG liposomes.

The drug is selected from Albuterol (salbutamol), Alfuzosin, Amantadine, Amiodarone, Amisulpride, Amitriptyline, Amoxapine, Amphetamine, Anagrelide, Apomorphine, Arformoterol, Aripiprazole, Arsenic trioxide, Astemizole, Atazanavir, Atomoxetine, Azithromycin, Bedaquiline, Bepridil, Bortezomib, Bosutinib, Chloral hydrate, Chloroquine, Chlorpromazine, Ciprofloxacin, Cisapride, Citalopram, Clarithromycin, Clomipramine, Clozapine, Cocaine, Crizotinib, Dabrafenib, Dasatinib, Desipramine, Dexmedetomidine, Dexmethylphenidate, Dextroamphetamine (d-Amphetamine), Dihydroartemisinin+piperaquine, Diphenhydramine, Disopyramide, Dobutamine, Dofetilide, Dolasetron, Domperidone, Dopamine, Doxepin, Dronedarone, Droperidol, Ephedrine, Epinephrine (Adrenaline), Eribulin, Erythromycin, Escitalopram, Famotidine, Felbamate, Fenfluramine, Fingolimod, Flecainide, Fluconazole, Fluoxetine, Formoterol, Foscarnet, Fosphenytoin, Furosemide (Frusemide), Galantamine, Gatifloxacin, Gemifloxacin, Granisetron, Halofantrine, Haloperidol, Hydrochlorothiazide, Ibutilide, Iloperidone, Imipramine (melipramine), Indapamide, Isoproterenol, Isradipine, Itraconazole, Ivabradine, Ketoconazole, Lapatinib, Levalbuterol (levsalbutamol), Levofloxacin, Levomethadyl, Lisdexamfetamine, Lithium, Mesoridazine, Metaproterenol, Methadone, Methamphetamine (methamfetamine), Methylphenidate, Midodrine, Mifepristone, Mirabegron, Mirtazapine, Moexipril/HCTZ, Moxifloxacin, Nelfinavir, Nicardipine, Nilotinib, Norepinephrine (noradrenaline), Norfloxacin, Nortriptyline, Ofloxacin, Olanzapine, Ondansetron, Oxytocin, Paliperidone, Paroxetine, Pasireotide, Pazopanib, Pentamidine, Perflutren lipid microspheres, Phentermine, Phenylephrine, Phenylpropanolamine, Pimozide, Posaconazole, Probucol, Procainamide, Promethazine, Protriptyline, Pseudoephedrine, Quetiapine, Quinidine, Quinine sulfate, Ranolazine, Rilpivirine, Risperidone, Ritodrine, Ritonavir, Roxithromycin, Salmeterol, Saquinavir, Sertindole, Sertraline, Sevoflurane, Sibutramine, Solifenacin, Sorafenib, Sotalol, Sparfloxacin, Sulpiride, Sunitinib, Tacrolimus, Tamoxifen, Telaprevir, Telavancin, Telithromycin, Terbutaline, Terfenadine, Tetrabenazine, Thioridazine, Tizanidine, Tolterodine, Toremifene, Trazodone, Trimethoprim-Sulfa, Trimipramine, Vandetanib, Vardenafil, Vemurafenib, Venlafaxine, Voriconazole, Vorinostat, or Ziprasidone.

In one embodiment, the present invention includes a composition for use in preventing or treating diseases with an active agent or drug that causes one or more adverse reactions arising from administration of an active agent or drug in a human that causes at least one of cardiac channelopathies, I_{Kr} channel inhibition or QT prolongation comprising:
an amount of a lysophosphatidylglycerol with a basic structure: 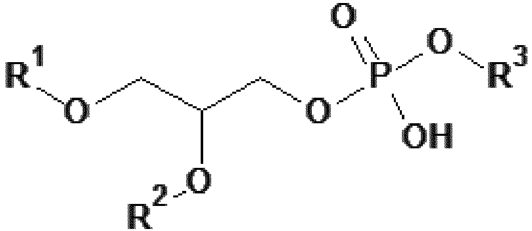
wherein R¹ or R² can be any even or odd-chain fatty acid, and R³ can be H, acyl, alkyl, aryl, amino acid, alkenes, alkynes, adapted for oral administration effective to reduce or prevent the at least one cardiac channelopathies, I_{Kr} channel inhibition or QT prolongation caused by the drug; and one or more active agents or drugs that cause at least one of I_{Kr} channel inhibition or QT prolongation and one or more thixotropic agent, or both organoleptic and thixotropic agents. In one aspect, the organoleptic agents include one or more flavorants, sweeteners, coolants, dyes, or combinations and mixtures thereof. Moreover, it has been found that in a powder dried according to the invention undesired organoleptic changes have been hardly effected, if effected at all, and that a powder dried according to the invention has sufficient solubility for various applications. The thixotropic agent forms a thixotrophic matrix, e.g., polysaccharides such as cellulose (e.g., carboxymethylcellulose) or gums (e.g., xanthan), collagen, gelatin, aerogels, polyacrylamide, alkyd resins, and silica-lipids. In one aspect, the composition includes both organoleptic and thixotropic agents. In one aspect, the phosphatidylglycerol is provided in the form of empty liposomes with a diameter of 10, 20, 25, 30, 40, 50, 60, 75, 80, 90, or 100 nM, e.g., 1-Myristoyl-2-Hydroxy-sn-Glycero-3-Phosphocholine (DMPC), 12-Mysteroyl-2-Hydroxy-sn-Glycero-3-[Phospho-rac-(glycerol)] (DMPG), or DMPC/DMPG liposomes. The lysophosphatidylglycerol includes at least one of a lysophosphatidylcholine, lauroyl-lysophosphatidylcholine, myristoyl-lysophosphatidylcholine, palmitoyl-lysophosphatidylcholine, stearoyl-lysophosphatidylcholine, arachidoyl-lysophosphatidylcholine, oleoyl-lysophosphatidylcholine, linoleoyl-lysophosphatidylcholine, linolenoyl-lysophosphatidylcholine or erucoyl-lysophosphatidylcholine. In another aspect, the liposome or liposome precursors are selected from at least one or 1-Myristoyl-2-Hydroxy-sn-Glycero-3-Phosphocholine (DMPC), 12-Mysteroyl-2-Hydroxy-sn-Glycero-3-[Phospho-rac-(glycerol)] (DMPG), DMPC/DMPG, 1-myristoyl-2-hydroxy-*sn*-glycero-3-phospho-(1'-*rac*-glycerol) (LysoPG), or 1-myristoyl-2-hydroxy-sn-glycero-3-phosphocholine (LysoPC). In another aspect, the short chain fatty acid is up to 5 carbons, a medium chain is 6 to 12 carbons, a long chain is 13-21 carbons and a very long chain fatty acid is greater than 22 carbons, including both even and odd chain fatty acids. In another aspect, the short chain fatty acid has 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55 or more carbons, which are saturated or unsaturated. In another aspect, the cardiac channelopathy or the condition resulting from the irregularity or alteration in the cardiac pattern is inhibition of an ion channel responsible for the delayed-rectifier K⁺ current in the heart, polymorphic ventricular tachycardia, prolongation of the QTc, LQT2, LQTS, or torsades de pointes. In another aspect, the composition is used for the treatment or prevention of prolongation of the I_{Kr} channel inhibition or QT prolongation induced by administration of one or more drugs used in the treatment of cardiac, allergic, or cancer related disease. In another aspect, the one or more active agents is selected from at least one of crizotinib, nilotinib, terfenadine, astemizole, gripafloxacin, terodilene, droperidole, lidoflazine, levomethady1, sertindoyle or cisapride. In another aspect, the active agent or drug is provided enterally, parenterally, intravenously, intraperitoneally, or orally. In another aspect, the liposomes comprises a lipid or a phospholipid wall, wherein the lipids or the phospholipids are selected from the group consisting of phosphatidylcholine (lecithin), lysolecithin, lysophosphatidylethanol-amine, phosphatidylserine, phosphatidylinositol, sphingomyelin, phosphatidylethanolamine (cephalin), cardiolipin, phosphatidic acid, cerebrosides, dicetylphosphate, phosphatidylcholine, and dipalmitoyl-phosphatidylglycerol, stearylamine, dodecylamine, hexadecyl-amine, acetyl palmitate, glycerol ricinoleate, hexadecyl sterate, isopropyl myristate, amphoteric acrylic polymers, fatty acid, fatty acid amides, cholesterol, cholesterol ester, diacylglycerol, and diacylglycerolsuccinate. The drug is selected from Albuterol (salbutamol), Alfuzosin, Amantadine, Amiodarone, Amisulpride, Amitriptyline, Amoxapine, Amphetamine, Anagrelide, Apomorphine, Arformoterol, Aripiprazole, Arsenic trioxide, Astemizole, Atazanavir, Atomoxetine, Azithromycin, Bedaquiline, Bepridil, Bortezomib, Bosutinib, Chloral hydrate, Chloroquine, Chlorpromazine, Ciprofloxacin, Cisapride, Citalopram, Clarithromycin, Clomipramine, Clozapine, Cocaine, Crizotinib, Dabrafenib, Dasatinib, Desipramine, Dexmedetomidine, Dexmethylphenidate, Dextroamphetamine (d-Amphetamine), Dihydroartemisinin+piperaquine, Diphenhydramine, Disopyramide, Dobutamine, Dofetilide, Dolasetron, Domperidone, Dopamine, Doxepin, Dronedarone, Droperidol, Ephedrine, Epinephrine (Adrenaline), Eribulin, Erythromycin, Escitalopram, Famotidine, Felbamate, Fenfluramine, Fingolimod, Flecainide, Fluconazole, Fluoxetine, Formoterol, Foscarnet, Fosphenytoin, Furosemide (Frusemide), Galantamine, Gatifloxacin, Gemifloxacin, Granisetron, Halofantrine, Haloperidol, Hydrochlorothiazide, Ibutilide, Iloperidone, Imipramine (melipramine), Indapamide, Isoproterenol, Isradipine, Itraconazole, Ivabradine, Ketoconazole, Lapatinib, Levalbuterol (levsalbutamol), Levofloxacin, Levomethadyl, Lisdexamfetamine, Lithium, Mesoridazine, Metaproterenol, Methadone, Methamphetamine (methamfetamine), Methylphenidate, Midodrine, Mifepristone, Mirabegron, Mirtazapine, Moexipril/HCTZ, Moxifloxacin, Nelfinavir, Nicardipine, Nilotinib, Norepinephrine (noradrenaline), Norfloxacin, Nortriptyline, Ofloxacin, Olanzapine, Ondansetron, Oxytocin, Paliperidone, Paroxetine, Pasireotide, Pazopanib, Pentamidine, Perflutren lipid microspheres, Phentermine, Phenylephrine, Phenylpropanolamine, Pimozide, Posaconazole, Probucol, Procainamide, Promethazine, Protriptyline, Pseudoephedrine, Quetiapine, Quinidine, Quinine sulfate, Ranolazine, Rilpivirine, Risperidone, Ritodrine, Ritonavir, Roxithromycin, Salmeterol, Saquinavir, Sertindole, Sertraline, Sevoflurane, Sibutramine, Solifenacin, Sorafenib, Sotalol, Sparfloxacin, Sulpiride, Sunitinib, Tacrolimus, Tamoxifen, Telaprevir, Telavancin, Telithromycin, Terbutaline, Terfenadine, Tetrabenazine, Thioridazine, Tizanidine, Tolterodine, Toremifene, Trazodone, Trimethoprim-Sulfa, Trimipramine, Vandetanib, Vardenafil, Vemurafenib, Venlafaxine, Voriconazole, Vorinostat, or Ziprasidone.

In one embodiment, the present invention includes a composition for use in preventing or treating one or more cardiac diseases in a human or animal subject caused by an active agent or drug, wherein the active agent or drug are used to treat a disease in a human or animal subject, the composition comprising: an amount of a lysophosphatidylglycerol adapted for oral administration effective to reduce or prevent the one or more cardiac diseases selected from channelopathies, irregularities or alterations in cardiac patterns, IKr channel inhibition, or QT prolongation caused by the active agent or drug; an effective amount of the active agent or drug sufficient to treat the one or more cardiac disease, wherein the orally provided lysophosphatidylglycerol reduces or eliminates the at least one or more cardiac diseases; and one or more thixotropic agent, or both organoleptic and thixotropic agents, wherein the composition is a liquid and the thixotropic agent forms a thixotrophic matrix and is selected from at least one of polysaccharides, cellulose, carboxymethylcellulose, gums, xanthan gum, collagen, gelatin, aerogels, polyacrylamide, alkyd resins, or silica-lipids, wherein the drug is selected from Albuterol (salbutamol), Alfuzosin, Amantadine, Amiodarone, Amisulpride, Amitriptyline, Amoxapine, Amphetamine, Anagrelide, Apomorphine, Arformoterol, Aripiprazole, Arsenic trioxide, Astemizole, Atazanavir, Atomoxetine, Azithromycin, Bedaquiline, Bepridil, Bortezomib, Bosutinib, Chloral hydrate, Chloroquine, Chlorpromazine, Ciprofloxacin, Cisapride, Citalopram, Clarithromycin, Clomipramine, Clozapine, Cocaine, Crizotinib, Dabrafenib, Dasatinib, Desipramine, Dexmedetomidine, Dexmethylphenidate, Dextroamphetamine (d-Amphetamine), Dihydroartemisinin+piperaquine, Diphenhydramine, Disopyramide, Dobutamine, Dofetilide, Dolasetron, Domperidone, Dopamine, Doxepin, Dronedarone, Droperidol, Ephedrine, Epinephrine (Adrenaline), Eribulin, Erythromycin, Escitalopram, Famotidine, Felbamate, Fenfluramine, Fingolimod, Flecainide, Fluconazole, Fluoxetine, Formoterol, Foscarnet, Fosphenytoin, Furosemide (Frusemide), Galantamine, Gatifloxacin, Gemifloxacin, Granisetron, Halofantrine, Haloperidol, Hydrochlorothiazide, Ibutilide, Iloperidone, Imipramine (melipramine), Indapamide, Isoproterenol, Isradipine, Itraconazole, Ivabradine, Ketoconazole, Lapatinib, Levalbuterol (levsalbutamol), Levofloxacin, Levomethadyl, Lisdexamfetamine, Lithium, Mesoridazine, Metaproterenol, Methadone, Methamphetamine (methamfetamine), Methylphenidate, Midodrine, Mifepristone, Mirabegron, Mirtazapine, Moexipril/HCTZ, Moxifloxacin, Nelfinavir, Nicardipine, Nilotinib, Norepinephrine (noradrenaline), Norfloxacin, Nortriptyline, Ofloxacin, Olanzapine, Ondansetron, Oxytocin, Paliperidone, Paroxetine, Pasireotide, Pazopanib, Pentamidine, Perflutren lipid microspheres, Phentermine, Phenylephrine, Phenylpropanolamine, Pimozide, Posaconazole, Probucol, Procainamide, Promethazine, Protriptyline, Pseudoephedrine, Quetiapine, Quinidine, Quinine sulfate, Ranolazine, Rilpivirine, Risperidone, Ritodrine, Ritonavir, Roxithromycin, Salmeterol, Saquinavir, Sertindole, Sertraline, Sevoflurane, Sibutramine, Solifenacin, Sorafenib, Sotalol, Sparfloxacin, Sulpiride, Sunitinib, Tacrolimus, Tamoxifen, Telaprevir, Telavancin, Telithromycin, Terbutaline, Terfenadine, Tetrabenazine, Thioridazine, Tizanidine, Tolterodine, Toremifene, Trazodone, Trimethoprim-Sulfa, Trimipramine, Vandetanib, Vardenafil, Vemurafenib, Venlafaxine, Voriconazole, Vorinostat, or Ziprasidone; and wherein the phosphatidylglycerol is selected from lysophosphatidylcholine, lauroyl-lysophosphatidylcholine, myristoyl-lysophosphatidylcholine, palmitoyl-lysophosphatidylcholine, stearoyl-lysophosphatidylcholine, arachidoyl-lysophosphatidylcholine, oleoyl-lysophosphatidylcholine, linoleoyl-lysophosphatidylcholine, linolenoyl-lysophosphatidylcholine or erucoyl-lysophosphatidylcholine.

In one aspect, the organoleptic agents include one or more flavorants, sweeteners, coolants, dyes, or combinations and mixtures thereof. Moreover, it has been found that in a powder dried according to the invention undesired organoleptic changes have been hardly effected, if effected at all, and that a powder dried according to the invention has sufficient solubility for various applications. The thixotropic agent forms a thixotrophic matrix, e.g., polysaccharides such as cellulose (e.g., carboxymethylcellulose) or gums (e.g., xanthan), collagen, gelatin, aerogels, polyacrylamide, alkyd resins, and silica-lipids. In one aspect, the composition includes both organoleptic and thixotropic agents. In one aspect, the phosphatidylglycerol is provided in the form of empty liposomes with a diameter of 10, 20, 25, 30, 40, 50, 60, 75, 80, 90, or 100 nM, e.g., 1-Myristoyl-2-Hydroxy-sn-Glycero-3-Phosphocholine (DMPC), 12-Mysteroyl-2-Hydroxy-sn-Glycero-3-[Phospho-rac-(glycerol)] (DMPG), or DMPC/DMPG liposomes. The lysophosphatidylglycerol includes at least one of a lysophosphatidylcholine, lauroyl-lysophosphatidylcholine, myristoyl-lysophosphatidylcholine, palmitoyl-lysophosphatidylcholine, stearoyl-lysophosphatidylcholine, arachidoyl-lysophosphatidylcholine, oleoyl-lysophosphatidylcholine, linoleoyl-lysophosphatidylcholine, linolenoyl-lysophosphatidylcholine or erucoyl-lysophosphatidylcholine. In another aspect, the liposome or liposome precursor are selected from at least one or 1-Myristoyl-2-Hydroxy-sn-Glycero-3-Phosphocholine (DMPC), 12-Mysteroyl-2-Hydroxy-sn-Glycero-3-[Phospho-rac-(glycerol)] (DMPG), DMPC/DMPG, 1-myristoyl-2-hydroxy-*sn*-glycero-3-phospho-(1'-*rac*-glycerol) (LysoPG), or 1-myristoyl-2-hydroxy-sn-glycero-3-phosphocholine (LysoPC). In another aspect, the short chain fatty acid is up to 5 carbons, a medium chain is 6 to 12 carbons, a long chain is 13-21 carbons and a very long chain fatty acid is greater than 22 carbons, including both even and odd chain fatty acids. In another aspect, the short chain fatty acid has 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55 or more carbons, which are saturated or unsaturated. In another aspect, the cardiac channelopathy or the condition resulting from the irregularity or alteration in the cardiac pattern is inhibition of an ion channel responsible for the delayed-rectifier K⁺ current in the heart, polymorphic ventricular tachycardia, prolongation of the QTc, LQT2, LQTS, or torsades de pointes. The one or more active agents is selected from at least one of crizotinib, nilotinib, terfenadine, astemizole, gripafloxacin, terodilene, droperidole, lidoflazine, levomethadyl, sertindoyle or cisapride. In another aspect, the drug is selected from Albuterol (salbutamol), Alfuzosin, Amantadine, Amiodarone, Amisulpride, Amitriptyline, Amoxapine, Amphetamine, Anagrelide, Apomorphine, Arformoterol, Aripiprazole, Arsenic trioxide, Astemizole, Atazanavir, Atomoxetine, Azithromycin, Bedaquiline, Bepridil, Bortezomib, Bosutinib, Chloral hydrate, Chloroquine, Chlorpromazine, Ciprofloxacin, Cisapride, Citalopram, Clarithromycin, Clomipramine, Clozapine, Cocaine, Crizotinib, Dabrafenib, Dasatinib, Desipramine, Dexmedetomidine, Dexmethylphenidate, Dextroamphetamine (d-Amphetamine), Dihydroartemisinin+piperaquine, Diphenhydramine, Disopyramide, Dobutamine, Dofetilide, Dolasetron, Domperidone, Dopamine, Doxepin, Dronedarone, Droperidol, Ephedrine, Epinephrine (Adrenaline), Eribulin, Erythromycin, Escitalopram, Famotidine, Felbamate, Fenfluramine, Fingolimod, Flecainide, Fluconazole, Fluoxetine, Formoterol, Foscarnet, Fosphenytoin, Furosemide (Frusemide), Galantamine, Gatifloxacin, Gemifloxacin, Granisetron, Halofantrine, Haloperidol, Hydrochlorothiazide, Ibutilide, Iloperidone, Imipramine (melipramine), Indapamide, Isoproterenol, Isradipine, Itraconazole, Ivabradine, Ketoconazole, Lapatinib, Levalbuterol (levsalbutamol), Levofloxacin, Levomethadyl, Lisdexamfetamine, Lithium, Mesoridazine, Metaproterenol, Methadone, Methamphetamine (methamfetamine), Methylphenidate, Midodrine, Mifepristone, Mirabegron, Mirtazapine, Moexipril/HCTZ, Moxifloxacin, Nelfinavir, Nicardipine, Nilotinib, Norepinephrine (noradrenaline), Norfloxacin, Nortriptyline, Ofloxacin, Olanzapine, Ondansetron, Oxytocin, Paliperidone, Paroxetine, Pasireotide, Pazopanib, Pentamidine, Perflutren lipid microspheres, Phentermine, Phenylephrine, Phenylpropanolamine, Pimozide, Posaconazole, Probucol, Procainamide, Promethazine, Protriptyline, Pseudoephedrine, Quetiapine, Quinidine, Quinine sulfate, Ranolazine, Rilpivirine, Risperidone, Ritodrine, Ritonavir, Roxithromycin, Salmeterol, Saquinavir, Sertindole, Sertraline, Sevoflurane, Sibutramine, Solifenacin, Sorafenib, Sotalol, Sparfloxacin, Sulpiride, Sunitinib, Tacrolimus, Tamoxifen, Telaprevir, Telavancin, Telithromycin, Terbutaline, Terfenadine, Tetrabenazine, Thioridazine, Tizanidine, Tolterodine, Toremifene, Trazodone, Trimethoprim-Sulfa, Trimipramine, Vandetanib, Vardenafil, Vemurafenib, Venlafaxine, Voriconazole, Vorinostat, or Ziprasidone.

In one embodiment, the present invention includes a composition for use in preventing or treating one or more adverse reactions arising from administration of a therapeutically active agent or a drug in a human or animal subject comprising the steps of administering to the human or animal subject an amount of an amount of a lysophosphatidylglycerol with a basic structure: 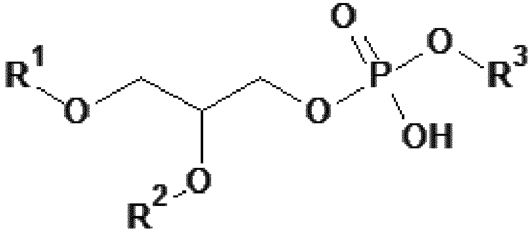 wherein R¹ or R² can be any even or odd-chain fatty acid, and R³ can be H, acyl, alkyl, aryl, amino acid, alkenes, alkynes, adapted for oral administration effective to reduce or prevent the at least one cardiac channelopathies, I_{Kr} channel inhibition or QT prolongation caused by the drug; and adapted for oral administration effective to reduce or prevent one or more cardiac channelopathies or conditions resulting from irregularities or alterations in cardiac patterns caused by the drug; and measuring the effect of the combination of the lysophosphatidylglycerol and the therapeutically active agent or the drug on the drug-induced channelopathy, wherein the composition reduces or eliminated the channelopathy induced by the therapeutically active agent or the drug, and one or more thixotropic agent, or both organoleptic and thixotropic agents. In one aspect, the organoleptic agents include one or more flavorants, sweeteners, coolants, dyes, or combinations and mixtures thereof. Moreover, it has been found that in a powder dried according to the invention undesired organoleptic changes have been hardly effected, if effected at all, and that a powder dried according to the invention has sufficient solubility for various applications. The thixotropic agent forms a thixotrophic matrix, e.g., polysaccharides such as cellulose (e.g., carboxymethylcellulose) or gums (e.g., xanthan), collagen, gelatin, aerogels, polyacrylamide, alkyd resins, and silica-lipids. In one aspect, the composition includes both organoleptic and thixotropic agents. In one aspect, the phosphatidylglycerol is provided in the form of empty liposomes with a diameter of 10, 20, 25, 30, 40, 50, 60, 75, 80, 90, or 100 nM, e.g., 1-Myristoyl-2-Hydroxy-sn-Glycero-3-Phosphocholine (DMPC), 12-Mysteroyl-2-Hydroxy-sn-Glycero-3-[Phospho-rac-(glycerol)] (DMPG), or DMPC/DMPG liposomes.

### BRIEF DESCRIPTION OF THE DRAWINGS

For a more complete understanding of the features and advantages of the present invention, reference is now made to the detailed description of the invention along with the accompanying figures and in which:
Figure 1 is a graph that shows the effect of DMPC, DMPC + Nilotinib and Nilotinib on hERG current density from transfected HEK 293 cells.
Figure 2 is a graph that shows the effect of DMPG, DMPG + Nilotinib and Nilotinib on hERG current density from transfected HEK 293 cells.
Figure 3 is a graph that shows the effect of DMPC/DMPG, DMPC/DMPG + Nilotinib and Nilotinib on hERG current density from transfected HEK 293 cells.
Figure 4 is a graph that shows the effect of LysoPC, LysoPC + Nilotinib and Nilotinib on hERG current density from transfected HEK 293 cells.
Figure 5 is a graph that shows the effect of LysoPG, LysoPG + Nilotinib and Nilotinib on hERG current density from transfected HEK 293 cells.
Figure 6 is a graph that shows the effect of DMPC, DMPC + Nilotinib, DMPC + Nilotinib (in DMSO) and Nilotinib on hERG current density from transfected HEK 293 cells.
Figure 7 is a graph that shows the effect of DMPG, DMPG + Nilotinib, DMPG + Nilotinib (in DMSO) and Nilotinib on hERG current density from transfected HEK 293 cells.

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

As used herein, the term "thixotropic" is used to describe one or more agents, e.g., certain gels, which liquefy when subjected to vibratory forces like simple shaking, and then solidify again when left standing. Thixotropic behavior is observed when long-chain molecules tend to orient themselves in the direction of flow; as the applied force is increased, the resistance to flow is decreased. Yet when high shear stress is removed, the solution will quickly revert to its original viscous state. Some celluloses exhibit thixotropic behavior wherein the solution returns to its viscous state over a period of time. Examples of thixotropic agents for use with, e.g., food, pharmaceuticals, are well known in the art, e.g., "A time-dependent expression for thixotropic areas. Application to Aerosil 200 hydrogels," M. Dolz, F. Gonzalez, J. Delegido, M. J. Hernandez, J. Pellicer, J. Pharm. Sci., Vol. 89, No. 6, pages 790-797 (2000). Numerous examples of thixotropic agents, such as cellulose (e.g., carboxymethylcellulose), gums (e.g., xanthan), collagen, gelatin, aerogels and others are well known in the art and may be used with the present invention, e.g., U.S. Pat. Nos. 6,709,675; 6,838,449; 6,818,018.

As used herein, an "organoleptic agent" refers to an additive with sensory attributes of a food or beverage, in particular the oral compositions provided herein. Those of skill in the art understand such properties and they can be quantitated if needed. Organoleptic properties include, but are not limited to, taste, odor and/or appearance. "Desirable" organoleptic properties include those organoleptic properties that make a food or beverage composition desirable for consumption by an average human subject, such as a desirable odor, taste and/or appearance, or the lack of an undesirable odor, taste and/or appearance. Undesirable organoleptic properties include the presence of, for example, an undesirable taste, odor or appearance attribute, such as the presence of an "off-taste" or "off-odor," for example a fishy, grassy, metal or iron, sharp or tingling taste or odor, or the presence of an undesirable appearance attribute, such as separation or precipitation. In one example, the provided beverage compositions retain the same or about the same taste, odor and/or appearance as the same beverage composition that does not contain the provided concentrates, that is, the provided beverage compositions retain organoleptic properties desirable for consumption by an average human subject. Desirable and undesirable organoleptic properties can be measured by a variety of methods known to those skilled in the art, including, for example, organoleptic evaluation methods by which undesirable properties are detectable by sight, taste and/or smell and chemical tests, as well as by chemical analytical methods. For example, the provided beverage compositions retain the same or about the same organoleptic properties as the same beverage composition that does not contain the provided concentrates over a period of time, for example, at least or over 1, 2, 3, 4, 5, 6, or more days, at least or over 1, 2, 3, 4, or more weeks, at least or over 1, 2, 3, 4, 5, 6, 7, 8, 9, 10, 11, 12, or more months, or at least or over 1, 2, 3, 4, or more years. As used herein, "retaining the organoleptic properties" refers to retention of these properties upon storage for a recited period of time, typically at room temperature.

Examples of suitable liquid dosage forms include solutions or suspensions in water, pharmaceutically acceptable fats and oils, alcohols or other organic solvents, including esters, emulsions, syrups or elixirs, suspensions, solutions and/or suspensions reconstituted from non-effervescent granules and effervescent preparations reconstituted from effervescent granules. Such liquid dosage forms may contain, for example, suitable solvents, preservatives, emulsifying agents, suspending agents, diluents, sweeteners, thickeners, and melting agents. Oral dosage forms optionally contain flavorants and coloring agents. Lysophosphatidylglycerols for use with the present invention include lysophosphatidylcholines, lauroyl-lysophosphatidylcholine, myristoyl-lysophosphatidylcholine, palmitoyl-lysophosphatidylcholine, stearoyl-lysophosphatidylcholine, arachidoyl-lysophosphatidylcholine, oleoyl-lysophosphatidylcholine, linoleoyl-lysophosphatidylcholine, linolenoyl-lysophosphatidylcholine or erucoyl-lysophosphatidylcholine. Asymmetric phosphatidylcholines are referred to as 1-acyl, 2-acyl-sn-glycero-3-phosphocholines, wherein the acyl groups are different from each other. Symmetric phosphatidylcholines are referred to as 1,2-diacyl-sn-glycero-3-phosphocholines. As used herein, the abbreviation "PC" refers to phosphatidylcholine. The phosphatidylcholine 1,2-dimyristoyl-sn-glycero-3-phosphocholine is abbreviated herein as "DMPC." The phosphatidylcholine 1,2-dioleoyl-sn-glycero-3-phosphocholine is abbreviated herein as "DOPC." The phosphatidylcholine 1,2-dipalmitoyl-sn-glycero-3-phosphocholine is abbreviated herein as "DPPC." The single fatty acid chain version of these short or long chain fatty acids are referred to as the "lyso" forms when only a single fatty acid chain is attached to the glyceryl backbone. In certain non-limiting examples, the phosphatidylglycerols form liposomes that are empty and have a diameter of 10, 20, 25, 30, 40, 50, 60, 75, 80, 90, or 100 nM.

As used herein, the term "additive" refers to a food, beverage, or other human consumable that enhances one or more of its nutritional, pharmaceutical, dietary, health, nutraceutical, health benefit, energy-providing, treating, holistic, or other properties such as dosing compliance. In certain embodiments of the present invention users of the composition will need one or more additional nutrients with the present invention. For example, the additives can be oil-based additives (e.g., non-polar compounds), such as nutraceuticals; pharmaceuticals; vitamins, for example, oil-soluble vitamins, e.g., vitamin D, vitamin E and vitamin A; minerals; fatty acids, such as essential fatty acids, for example, polyunsaturated fatty acids, e.g., omega-3 fatty acids and omega-6 fatty acids, such as alpha-linolenic acid (ALA), docosahexaenoic acid (DHA), eicosapentaenoic acid (EPA), gamma-linolenic acid GLA, CLA, saw palmetto extract, flaxseed oil, fish oil and algae oil; phytosterols; coenzymes, such as coenzyme Q10; and any other oil-based additives. Furthermore, in certain embodiments, the composition may have reduced dosing compliance as a result of the taste or smell of the active agents and/or the phosphatidylglycerol.

In one embodiment, the lysophosphatidylglycerol has a basic structure: 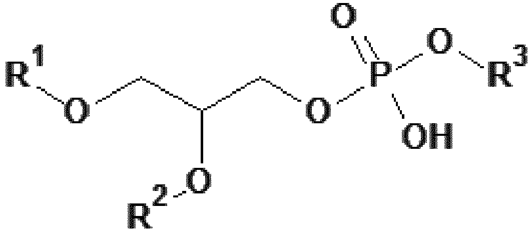 wherein R¹ or R² can be any even or odd-chain fatty acid, and R³ can be H, acyl, alkyl, aryl, amino acid, alkenes, alkynes, and wherein a short chain fatty acid is up to 5 carbons, a medium chain is 6 to 12 carbons, a long chain is 13-21 carbons and a very long chain fatty acid is greater than 22 carbons, including both even and odd chain fatty acids. In one example, the fatty acids have 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55 or long fatty acids, which can be saturated or unsaturated.

The present invention can be used with any QT prolonging drug, including Albuterol (salbutamol), Alfuzosin, Amantadine, Amiodarone, Amisulpride, Amitriptyline, Amoxapine, Amphetamine, Anagrelide, Apomorphine, Arformoterol, Aripiprazole, Arsenic trioxide, Astemizole, Atazanavir, Atomoxetine, Azithromycin, Bedaquiline, Bepridil, Bortezomib, Bosutinib, Chloral hydrate, Chloroquine, Chlorpromazine, Ciprofloxacin, Cisapride, Citalopram, Clarithromycin, Clomipramine, Clozapine, Cocaine, Crizotinib, Dabrafenib, Dasatinib, Desipramine, Dexmedetomidine, Dexmethylphenidate, Dextroamphetamine (d-Amphetamine), Dihydroartemisinin+piperaquine, Diphenhydramine, Disopyramide, Dobutamine, Dofetilide, Dolasetron, Domperidone, Dopamine, Doxepin, Dronedarone, Droperidol, Ephedrine, Epinephrine (Adrenaline), Eribulin, Erythromycin, Escitalopram, Famotidine, Felbamate, Fenfluramine, Fingolimod, Flecainide, Fluconazole, Fluoxetine, Formoterol, Foscarnet, Fosphenytoin, Furosemide (Frusemide), Galantamine, Gatifloxacin, Gemifloxacin, Granisetron, Halofantrine, Haloperidol, Hydrochlorothiazide, Ibutilide, Iloperidone, Imipramine (melipramine), Indapamide, Isoproterenol, Isradipine, Itraconazole, Ivabradine, Ketoconazole, Lapatinib, Levalbuterol (levsalbutamol), Levofloxacin, Levomethadyl, Lisdexamfetamine, Lithium, Mesoridazine, Metaproterenol, Methadone, Methamphetamine (methamfetamine), Methylphenidate, Midodrine, Mifepristone, Mirabegron, Mirtazapine, Moexipril/HCTZ, Moxifloxacin, Nelfinavir, Nicardipine, Nilotinib, Norepinephrine (noradrenaline), Norfloxacin, Nortriptyline, Ofloxacin, Olanzapine, Ondansetron, Oxytocin, Paliperidone, Paroxetine, Pasireotide, Pazopanib, Pentamidine, Perflutren lipid microspheres, Phentermine, Phenylephrine, Phenylpropanolamine, Pimozide, Posaconazole, Probucol, Procainamide, Promethazine, Protriptyline, Pseudoephedrine, Quetiapine, Quinidine, Quinine sulfate, Ranolazine, Rilpivirine, Risperidone, Ritodrine, Ritonavir, Roxithromycin, Salmeterol, Saquinavir, Sertindole, Sertraline, Sevoflurane, Sibutramine, Solifenacin, Sorafenib, Sotalol, Sparfloxacin, Sulpiride, Sunitinib, Tacrolimus, Tamoxifen, Telaprevir, Telavancin, Telithromycin, Terbutaline, Terfenadine, Tetrabenazine, Thioridazine, Tizanidine, Tolterodine, Toremifene, Trazodone, Trimethoprim-Sulfa, Trimipramine, Vandetanib, Vardenafil, Vemurafenib, Venlafaxine, Voriconazole, Vorinostat, or Ziprasidone.

Human ether-a-go-go-related gene (hERG) Potassium channel anti-blockade by liposome and fragments.

Potassium channels conduct the rapid component of the delayed rectifier potassium current, Kir, which is crucial for repolarization of cardiac action potentials. A reduction in hERG currents due to either genetic defects or adverse drug effects can lead to hereditary or acquired long QT syndromes characterized by action potential prolongation, lengthening of the QT interval on the surface ECG, and an increased risk for "torsade de pointes" arrhythmias and sudden death. This undesirable side effect of non-antiarrhythmic compounds has prompted the withdrawal of drugs from the market. Studies on mechanisms of hERG channel inhibition provide significant insights into the molecular factors that determine state-, voltage-, and use-dependency of hERG current block. Mutations altering properties of the high-affinity drug binding site in hERG and its interaction with drug molecules cause current increase and hereditary short QT syndrome with a high risk for life-threatening arrhythmias. (Thomas D1, 2006).

Anatomical Characteristics of the K+ channel. The types and distributions of inwardly rectifying potassium (Kir) channels are one of the major determinants of the electrophysiological properties of cardiac myocytes. Inward rectifier potassium (Kir) channels regulate cell excitability and transport of K+ ions across cell membranes.

The potassium channel from *Streptomyces lividans* is an integral membrane protein with sequence similarity to all known K⁺ channels, particularly in the pore region. X-ray analysis with data to 3.2 angstroms reveals that four identical subunits create an inverted teepee, or cone, cradling the selectivity filter of the pore in its outer end. The narrow selectivity filter is only 12 angstroms long, whereas the remainder of the pore is wider and lined with hydrophobic amino acids. A large water-filled cavity and helix dipoles are positioned so as to overcome electrostatic destabilization of an ion in the pore at the center of the bilayer. Main chain carbonyl oxygen atoms from the K⁺ channel signature sequence line the selectivity filter, which is held open by structural constraints to coordinate K⁺ ions but not smaller Na⁺ ions. The selectivity filter contains two K⁺ ions about 7.5 angstroms apart. Ion channels exhibit ion selectivity through pore architecture that conducts specific ions. This configuration promotes ion conduction by exploiting electrostatic repulsive forces to overcome attractive forces between K⁺ ions and the selectivity filter. The architecture of the pore establishes the physical principles underlying selective K⁺ conduction. (Doyle DA, 1998).

Another member of the inward-rectifier family of potassium channels is the prokaryotic KirBac 1.1 channel. The structure of the Kir channel assembly in the closed state, when refined to a resolution of 3.65 angstroms contains a main activation gate and structural elements involved in gating. On the basis of structural evidence, gating involves coupling between the intracellular and membrane domains suggesting that initiation of gating by membrane or intracellular signals represents different entry points to a common mechanistic pathway. (Kuo,A 2003).

Kir channels in the cardiac myocytes may be actively regulated by means of the change in PIP(2) level rather than by downstream signal transduction pathways. The classical inward rectifier K(+) channel), Kir2.1, Kir6.2/SUR2A (ATP-sensitive K(+) channel) and Kir3.1/3.4 (muscarinic K(+) channels) in cardiac myocytes are commonly upregulated by a membrane lipid, phosphatidylinositol 4,5-bisphosphates (PIP(2)). PIP(2) interaction sites appear to be conserved by positively charged amino acid residues and the putative alpha-helix in the C-terminals of Kir channels. PIP(2) level in the plasma membrane is regulated by tagonist stimulation (Takano MI 2003).

Inward rectifier potassium channels are characterized by two transmembrane helices per subunit, plus an intracellular C-terminal domain that controls channel gating in response to changes in concentration of various ligands. Based on the crystal structure of the tetrameric C-terminal domain of Kir3.1, it is possible to build a homology model of the ATP-binding C-terminal domain of Kir6.2. Molecular dynamics simulations are used to probe the dynamics of Kir C-terminal domains and to explore the relationship between their dynamics and possible mechanisms of channel gating. Multiple simulations, each of 10 ns duration, were performed for Kir3.1 (crystal structure) and Kir6.2 (homology model), in both their monomeric and tetrameric forms. The Kir6.2 simulations were performed with and without bound ATP. The results of the simulations reveal comparable conformational stability for the crystal structure and the homology model. There is decrease in conformational flexibility when comparing the monomers with the tetramers, corresponding mainly to the subunit interfaces in the tetramer. The beta-phosphate of ATP interacts with the side chain of K185 in the Kir6.2 model and simulations. The flexibility of the Kir6.2 tetramer is not changed greatly by the presence of bound ATP, other than in two loop regions. Principal components analysis of the simulated dynamics suggests loss of symmetry in both the Kir3.1 and Kir6.2 tetramers, consistent with "dimer-of-dimers" motion of subunits in C-terminal domains of the corresponding Kir channels. This is suggestive of a gating model in which a transition between exact tetrameric symmetry and dimer-of-dimers symmetry is associated with a change in transmembrane helix packing coupled to gating of the channel. Dimer-of-dimers motion of the C-terminal domain tetramer is also supported by coarse-grained (anisotropic network model) calculations. Loss of exact rotational symmetry is suggested to play a role in gating in the bacterial Kir homolog, KirBac1.1, and in the nicotinic acetylcholine receptor channel. (Haider SI, 2005).

Homotetrameric models of three mammalian Kir channels (Kir1.1, Kir3.1, and Kir6.2) have been generated, using the KirBac3.1 transmembrane and rat Kir3.1 intracellular domain structures as templates. All three models were explored by 10 ns molecular dynamics simulations in phospholipid bilayers. Analysis of the initial structures revealed conservation of potential lipid interaction residues (Trp/Tyr and Arg/Lys side chains near the lipid headgroup-water interfaces). Examination of the intracellular domains revealed key structural differences between Kir1.1 and Kir6.2 which may explain the difference in channel inhibition by ATP. The behavior of all three models in the MD simulations revealed that they have conformational stability similar to that seen for comparable simulations of, for example, structures derived from cryoelectron microscopy data. Local distortions of the selectivity filter were seen during the simulations, as observed in previous simulations of KirBac and in simulations and structures of KcsA. These may be related to filter gating of the channel. The intracellular hydrophobic gate does not undergo any substantial changes during the simulations and thus remains functionally closed. Analysis of lipid-protein interactions of the Kir models emphasizes the key role of the M0 (or "slide") helix which lies approximately parallel to the bilayer-water interface and forms a link between the transmembrane and intracellular domains of the channel (Haider SI, 2007).

The potassium-selective transmembrane pore in voltage-activated K+ channels is gated by changes in the membrane potential. Activation gating (opening) occurs in milliseconds and involves a gate at the cytoplasmic side of the pore. Substituting cysteine at a particular position in the last transmembrane region (S6) of the homotetrameric Shaker K+ channel creates metal binding sites at which Cd2+ ions can bind with high affinity. The bound Cd2+ ions form a bridge between the introduced cysteine in one channel subunit and a native histidine in another subunit, and the bridge traps the gate in the open state. These results suggest that gating involves a rearrangement of the intersubunit contacts at the intracellular end of S6. The structure of a bacterial K+ channel shows that the S6 homologs cross in a bundle, leaving an aperture at the bundle crossing. In the context of this structure, the metal ions form a bridge between a cysteine above the bundle crossing and a histidine below the bundle crossing in a neighboring subunit. results suggest that gating occurs at the bundle crossing, possibly through a change in the conformation of the bundle itself (Holmgren ML 2002).

Activated gating in voltage-activated K+ channels are a potassium-selective transmembrane pore gated by changes in the membrane potential. This activation gating (opening) occurs in milliseconds and involves a gate at the cytoplasmic side of the pore. Substituting cysteine at a particular position in the last transmembrane region (S6) of the homotetrameric Shaker K+ channel creates metal binding sites at which Cd2+ ions can bind with high affinity. The bound Cd2+ ions form a bridge between the introduced cysteine in one channel subunit and a native histidine in another subunit, and the bridge traps the gate in the open state. These results suggest that gating involves a rearrangement of the intersubunit contacts at the intracellular end of S6. The structure of a bacterial K+ channel shows that the S6 homologs cross in a bundle, leaving an aperture at the bundle crossing. In the context of this structure, the metal ions form a bridge between a cysteine above the bundle crossing and a histidine below the bundle crossing in a neighboring subunit. results suggest that gating occurs at the bundle crossing, possibly through a change in the conformation of the bundle itself (Holmgren ML 2002).

### Channelopathies

The human *ether-à-go-go* gene related cardiac tetrameric potassium channel. when mutated can render patients sensitive to over 163 drugs which may inhibit ion conduction and deregulate action potentials.(Credible Meds) Prolongation of the action potential follows effects in the potassium channel. Ion channel active drugs may directly increase the QTc interval, and increase the risk of torsade de point and sudden cardiac death.(Table 1) Exacerbation of cardiomyocyte potassium channel sensitivity to drugs may also be associated with metabolic diseased states including diabetes(Veglio M, 2002) or may be of idiopathic origin.

For these reasons, evaluation of drug effects on cardiomyocyte potassium channel function is a critical step during drug development, and when serious, may be an obstacle to regulatory approval. In whole-cell patch-clamp experiments, curcumin inhibited hERG K⁺ currents in HEK293 cells stably expressing hERG channels in a dose-dependent manner, with IC₅₀ value of 5.55 µM. The deactivation, inactivation and the recovery time from inactivation of hERG channels were significantly changed by acute treatment of 10 µM curcumin. Incubation of 20 µM curcumin for 24h reduced the HEK293 cell viability. Intravenous injection of 20 mg of curcumin in rabbits did not affect the cardiac repolarization manifested by QTc values. (Hu CW 2012). However, SignPath Pharma has discovered specific molecules which antagonize QTc prolonging drugs (Helson L, 2002 Ranjan A, 2014, Shopp G, 2014). These molecules are specific liposomes, or components of liposomes which were initially bound to lipophilic drugs to permit intravenous solubility at physiological conditions, and reduce adverse events. The loci of action appears to be in intra-channel ion selectivity or gating site(s) controlling potassium ion movement: a key functional component of regulation of action potentials which lead downstream to myocyte contraction.

The mechanism of human *ether-à-go-go* related gene channels blocade may be analogous to the effects of externally applied quaternary ammonium derivatives which indirectly may suggest the mechanism of action of the anti-blockading effect of the DMPC/DMPG liposome or its metabolites. The inhibitory constants and the relative binding energies for channel inhibition indicate that more hydrophobic quaternary ammoniums have higher affinity blockade while cation-π interactions or size effects are not a deterministic factor in channel inhibition by quaternary ammoniums. Also hydrophobic quaternary ammoniums either with a longer tail group or with a bigger head group than tetraethylammonium permeate the cell membrane to easily access the high-affinity internal binding site in the gene channel and exert a stronger blockade.

Although these data suggest that the basis for the ameliorating effect liposome, or its components is the higher competitive affinity for binding sites by the, DMPC and DMPG compared to QTc prolonging drugs( ), its constitutive lack of ion transport modulation, i.e. liposome or its fragments do not impede K+ ion transport indicates that

By way of explanation, and in no way a limitation of these claims, these data suggest that the basis for the ameliorating effect liposome, or its components, is the higher competitive affinity for binding sites by the DMPC and DMPG compared to QTc prolonging drugs, its constitutive lack of ion transport modulation, i.e., liposome, or its fragments, do not impede K+ ion transport and indicates that the site of the mechanism of DMPC or DMPG protection may be in the selectivity segment of the channel or in the hydration surrounding the ion.

Additionally, based upon these hERG channel data the structures of these liposome components may be informative for designing or selecting other molecules to prevent drug induced cardiac arrhythmias.

This study provides additional information as to the QTc modulating effects by drugs, induced in cardiac myocyte potassium channels, and mitigation by liposomes and liposomal constituents. The latter molecules present an opportunity to probe the K⁺ channels as targets for pharmacological mitigation of drug-induced channelopathies.

Evaluation of the protective effect of DMPC, DMPG, DMPC/DMPG, LysoPG and LysoPC against hERG inhibition by Nilotinib.

Purpose of the study: The purpose of this study is to evaluate *in vitro* the protective effect of DMPC, DMPG, DMPC/DMPG, LysoPG and LysoPC on the rapidly activating delayed-rectifier potassium selective current (I_{Kr}) generated under normoxic conditions in stably transfected Human Embryonic Kidney cells (HEK 293 cells). This study was designed as a screen and does not require QA involvement (non-GLP-compliant).

### Test Articles:

- 1-: DMPC
- 2-: DMPG
- 3-: DMPC/DMPG 90:9
- 4-: 14:0 LysoPC
- 5-: 14:0 LysoPG
- 6-: DMPC + Nilotinib (0.1 µM)
- 7-: DMPG + Nilotinib (0.1 µM)
- 8-: DMPC/DMPG 90:9 + Nilotinib (0.1 µM)
- 9-: 14:0 LysoPC + Nilotinib (0.1 µM)
- 10-: 14:0 LysoPG + Nilotinib (0.1 µM)

Test System: hERG-expressing HEK 293 transfected cell line. Test performed: Whole-cell patch-clamp current acquisition and analysis. Experimental Temperature: 35 ± 2°C.

### Application of test articles:

5 minutes of exposure to each concentration in presence of closed circuit perfusion (2 mL/min). 5 minutes for washout periods in presence of a flow-through perfusion (2 mL/min) in addition to a closed circuit perfusion (2 mL/min). The positive control (Nilotinib, 0.05 µg/mL) was added to naive cells obtained from the same cell line and same passage for a period of 5 minutes in presence of a closed circuit perfusion (2 mL/min).

Cells were under continuous stimulation of the pulses protocol throughout the experiments and cell currents were recorded after 5 minutes of exposure to each condition.

Original data acquisition design: Acquisition Rate(s): 1.0 kHz.

Design for acquisition when testing the compound or the vehicle/solvent equivalent:
1 recording made in baseline condition
1 recording made in the presence of concentration 1

Design for acquisition when testing the positive control:
1 recording made in baseline condition
1 recording made in the presence of the positive control
n = number of responsive cells patched on which the whole protocol above could be applied.

Statistical analysis: Statistical comparisons were made using paired Student's t-tests. The currents recorded obtained on day 2, 3 and 4 were statistically compared to the currents recorded on day 1.

The currents recorded after the positive control (nilotinib alone) exposure were compared to the currents recorded in baseline conditions.

Differences were considered significant when p ≤ 0.05.

### Exclusion criteria:

- 1.: Timeframe of drug exposure not respected
- 2.: Instability of the seal
- 3.: No tail current generated by the patched cell
- 4.: No significant effect of the positive control
- 5.: More than 10% variability in capacitance transient amplitude over the duration of the Study.

Effect of the Test Articles on whole-cell I_{Kr} hERG currents. Whole-cell currents elicited during a voltage pulse were recorded in baseline conditions and following the application of the selected concentration of test article. The cells were depolarized for one second from the holding potential (-80 mV) to a maximum value of +40 mV, starting at -40 mV and progressing in 10 mV increments. The membrane potential was then repolarized to -55 mV for one second, and finally returned to -80 mV.

Whole-cell tail current amplitude was measured at a holding potential of -55 mV, following activation of the current from -40 to +40 mV. Current amplitude was measured at the maximum (peak) of this tail current. Current density was obtained by dividing current amplitude by cell capacitance measured prior to capacitive transient minimization.

Current run-down and solvent effect correction. All data points presented in this Study Report have been corrected for solvent effect and time-dependent current run-down. Current run-down and solvent effects were measured simultaneously by applying the experimental design in test-article free conditions over the same time frame as was done with the test article. The loss in current amplitude measured during these so-called vehicle experiments (representing both solvent effects and time-dependent run-down) was subtracted from the loss of amplitude measured in the presence of the test article to isolate the effect of the test article, apart from the effect of the solvent and the inevitable run-down in current amplitude over time.

**Table 1. Effect of DMPC, DMPC + Nilotinib and Nilotinib on hERG current density from transfected HEK 293 cells.**

| | Normalized Current Density | Corrected Normalized Current Density | SEM | p value | n = |
|---|---|---|---|---|---|
| Baseline | 1.000 | 1.000 | n/a | n/a | 3 |
| DMPC | 0.863 | 1.056 | 0.056 | 0.423 | 3 |
| Nilotinib, 0.1 µM | 0.308 | 0.459* | 0.070 | 0.016 | 3 |
| DMPC + Nilotinib, 0.1 µM | 0.836 | 1.029 | 0.023 | 0.328 | 3 |

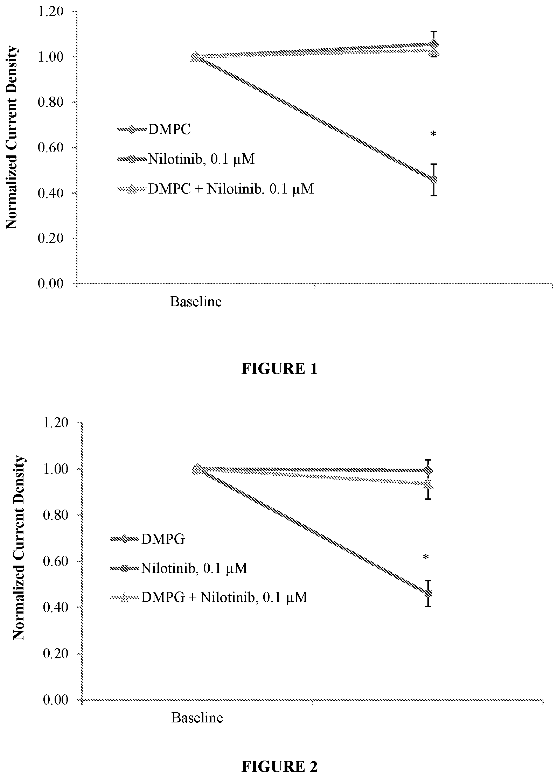

Figure 1 is a graph that shows the effect of DMPC, DMPC + Nilotinib and Nilotinib on hERG current density from transfected HEK 293 cells.

**Table 2. Effect of DMPG, DMPG + Nilotinib and Nilotinib on hERG current density from transfected HEK 293 cells.**

| | Normalized Current Density | Corrected Normalized Current Density | SEM | p value | n = |
|---|---|---|---|---|---|
| Baseline | 1.000 | 1.000 | n/a | n/a | 3 |
| DMPG | 0.800 | 0.994 | 0.044 | 0.901 | 3 |
| Nilotinib, 0.1 µM | 0.308 | 0.459* | 0.070 | 0.016 | 3 |
| DMPG + Nilotinib, 0.1 µM | 0.743 | 0.936 | 0.067 | 0.437 | 3 |

Figure 2 is a graph that shows the effect of DMPG, DMPG + Nilotinib and Nilotinib on hERG current density from transfected HEK 293 cells.

**Table 3. Effect of DMPC/DMPG, DMPC/DMPG + Nilotinib and Nilotinib on hERG current density from transfected HEK 293 cells.**

| | Normalized Current Density | Corrected Normalized Current Density | SEM | p value | n = |
|---|---|---|---|---|---|
| Baseline | 1.000 | 1.000 | n/a | n/a | 3 |
| DMPC-DMPG | 0.871 | 1.064 | 0.127 | 0.647 | 4 |
| Nilotinib, 0.1 µM | 0.308 | 0.459* | 0.070 | 0.016 | 3 |
| DMPC/DMPG + Nilotinib, 0.1 µM | 0.773 | 0.966 | 0.098 | 0.754 | 4 |

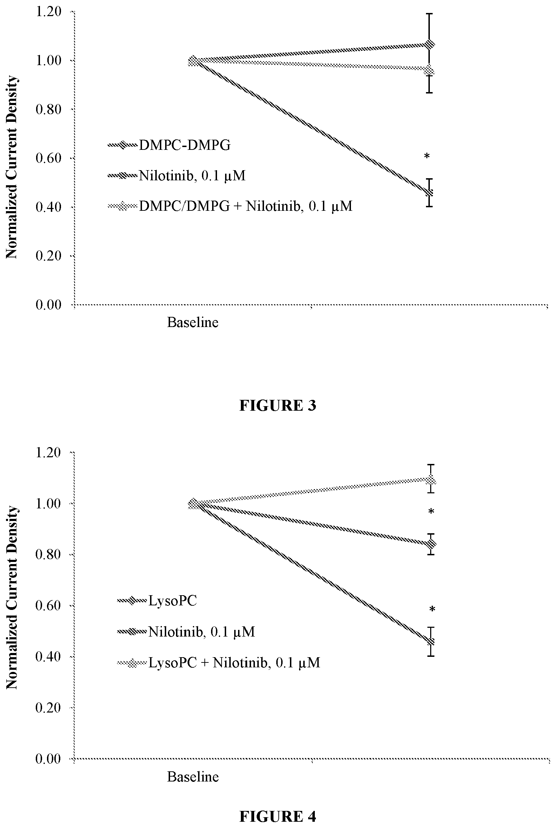

Figure 3 is a graph that shows the effect of DMPC/DMPG, DMPC/DMPG + Nilotinib and Nilotinib on hERG current density from transfected HEK 293 cells.

**Table 4. Effect of LysoPC, LysoPC + Nilotinib and Nilotinib on hERG current density from transfected HEK 293 cells.**

| | Normalized Current Density | Corrected Normalized Current Density | SEM | p value | n = |
|---|---|---|---|---|---|
| Baseline | 1.000 | 1.000 | n/a | n/a | 3 |
| LysoPC | 0.647 | 0.840* | 0.040 | 0.028 | 4 |
| Nilotinib, 0.1 µM | 0.308 | 0.459* | 0.070 | 0.016 | 3 |
| LysoPC + Nilotinib, 0.1 µM | 0.865 | 1.097 | 0.055 | 0.553 | 3 |

Figure 4 is a graph that shows the effect of LysoPC, LysoPC + Nilotinib and Nilotinib on hERG current density from transfected HEK 293 cells.

**Table 5. Effect of LysoPG, LysoPG + Nilotinib and Nilotinib on hERG current density from transfected HEK 293 cells.**

| | Normalized Current Density | Corrected Normalized Current Density | SEM | p value | n = |
|---|---|---|---|---|---|
| Baseline | 1.000 | 1.000 | n/a | n/a | 3 |
| 14:0 LysoPG, 0.45 µg/mL | 0.930 | 1.124 | 0.128 | 0.435 | 3 |
| Nilotinib, 0.1 µM | 0.308 | 0.459* | 0.070 | 0.016 | 3 |
| 14:0 LysoPG + Nilotinib, 0.1 µM | 0.743 | 0.936 | 0.067 | 0.437 | 3 |

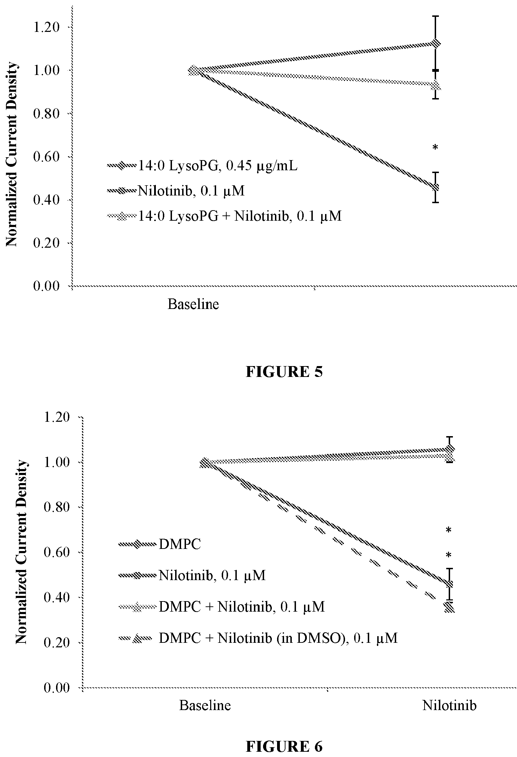

Figure 5 is a graph that shows the effect of LysoPG, LysoPG + Nilotinib and Nilotinib on hERG current density from transfected HEK 293 cells.

This study aimed at quantifying the protective effect of DMPC, DMPG, DMPC/DMPG, LysoPG and LysoPC against the inhibition of the rapidly activating delayed-rectifier potassium selective current (I_{Kr}) generated under normoxic conditions in stably transfected Human Embryonic Kidney (HEK) 293 cells caused by the Nilotinib.

All data points presented in this study have been corrected for solvent effects and time-dependent current run-down. These two parameters were evaluated by applying exactly the same experimental design to the vehicle as that done with the test articles. The currents were measured over the same time course as was done in the presence of the test article. The values obtained, representing both solvent effects and time-dependent run-down, were used to correct the effect of the test articles, if any. This ensures that changes attributable to time or the solvent are not mistakenly attributed to the test articles.

DMPC, DMPG, DMPC/DMPG and LysoPG alone did not cause any inhibition of the hERG tail current density (n=3). LysoPC alone caused 16% of inhibition of the hERG tail current density (n = 4).

Nilotinib alone, formulated in DMSO at 0.1 µM, caused 54.1% of inhibition of the hERG tail current (n = 3). The inhibition observed is in line with previous data generated in identical conditions, and agrees with reported inhibition values for this compound.

Nilotinib when formulated in an aqueous solution containing DMPC, DMPG, DMPC/DMPC, LysoPG or LysoPC (ratio 1:9) did not cause any inhibition of the hERG tail current.

These data suggest that co-formulating Nilotinib with DMPC, DMPG, DMPC/DMPC, LysoPG and LysoPC protects against hERG inhibition caused by Nilotinib.

In this study, the DMPC + Nilotinib, DMPG + Nilotinib, DMPC/DMPC + Nilotinib, LysoPG + Nilotinib or LysoPC + Nilotinib were all formulated using the same method. The appropriate amount of Nilotinib powder was dissolved in an aqueous solution containing either DMPC, DMPG, DMPC/DMPC, LysoPG or LysoPC (ratio 9:1). The solution was vortexed for 10 minutes before being used in the patch-clamp assay.

In contrast, the Nilotinib used for the cells exposed to Nilotinib alone was dissolved in DMSO. Additional studies were conducted to determine whether the difference in hERG inhibition between DMSO-formulated Nilotinib and lipid-co-formulated Nilotinib resulted from the different formulations (aqueous or DMSO-based).

### Steps for the study:

| Step 1 | Step 2 | Step 3 | Step 4 |
|---|---|---|---|
| Baseline recording | TA* added into the experimental chamber | 5 minutes exposure time | TA recording |

| | | | |
|---|---|---|---|
| *TA = | | | |

- 1-: DMPC (in aqueous solution)
- 2-: DMPG (in aqueous solution)
- 3-: DMPC/DMPG 90:9 (in aqueous solution)
- 4-: 14:0 LysoPC (in aqueous solution)
- 5-: 14:0 LysoPG( in aqueous solution)

- 6-: DMPC + Nilotinib (0.1 µM) (in aqueous solution)
- 7-: DMPG + Nilotinib (0.1 µM) (in aqueous solution)
- 8-: DMPC/DMPG 90:9 + Nilotinib (0.1 µM) (in aqueous solution)
- 9-: 14:0 LysoPC + Nilotinib (0.1 µM) (in aqueous solution)
- 10-: 14:0 LysoPG + Nilotinib (0.1 µM) (in aqueous solution)
- 11-: Nilotinib alone (in DMSO)

Amongst the mechanisms considered to explain the protection of hERG currents were the possibility that DMPC/DMPG or the Lyso- variants quenched the Nilotinib at the moment of formulation, essentially preventing it from getting into the channel at its receptor site. Another possibility was that Nilotinib was less soluble in an aqueous solution, and therefore was incompletely solubilized at 0.1 µM.

To test both hypotheses, Nilotinib was formulated in DMSO and added into the experimental chamber following the addition of the DMPC or DMPG. This was based on the principle that 1-adding DMPC/DMPG alone, followed by DMSO-formulated Nilotinib, would eliminate the possibility of early quenching of Nilotinib by the lysosome; and 2- that DMSO would maintain the solubility of Nilotinib (the "Nilotinib-only" inhibition of hERG was observed when DMSO-formulated Nilotinib was added to the cells).

### Steps for the following Data

| Step 1 | Step 2 | Step 3 | Step 4 | Step 5 | Step 6 |
|---|---|---|---|---|---|
| Baseline recording | DMPC or DMPG added into the experimental chamber | 5 minutes exposure time | DMPC or DMPG recording | Nilotinib in DMSO added into the experimental chamber | DMPC or DMPG + Nilotinib (in DMSO) recording |

**Table 6. Effect of DMPC, DMPC + Nilotinib, DMPC + Nilotinib (in DMSO) and Nilotinib on hERG current density from transfected HEK 293 cells.**

| | Normalized Current Density | Corrected Normalized Current Density | SEM | p value | n = |
|---|---|---|---|---|---|
| Baseline | 1.000 | 1.000 | n/a | n/a | 3 |
| DMPC | 0.863 | 1.056 | 0.056 | 0.423 | 3 |
| Nilotinib, 0.1 µM | 0.308 | 0.459* | 0.070 | 0.016 | 3 |
| DMPC + Nilotinib, 0.1 µM (Aqueous) | 0.836 | 1.029 | 0.023 | 0.328 | 3 |
| DMPC + Nilotinib (in DMSO), 0.1 µM | 0.164 | 0.358* | 0.020 | 0.019 | 2 |

Figure 6 is a graph that shows the effect of DMPC, DMPC + Nilotinib, DMPC + Nilotinib (in DMSO) and Nilotinib on hERG current density from transfected HEK 293 cells.

**Table 7. Effect of DMPG, DMPG + Nilotinib, DMPG + Nilotinib (in DMSO) and Nilotinib on hERG current density from transfected HEK 293 cells.**

| | Normalized Current Density | Corrected Normalized Current Density | SEM | p value | n = |
|---|---|---|---|---|---|
| Baseline | 1.000 | 1.000 | n/a | n/a | 3 |
| DMPG | 0.800 | 0.994 | 0.044 | 0.901 | 3 |
| Nilotinib, 0.1 µM | 0.308 | 0.459* | 0.070 | 0.016 | 3 |
| DMPG + Nilotinib, 0.1 µM | 0.743 | 0.936 | 0.067 | 0.437 | 3 |
| DMPG + Nilotinib (in DMSO), 0.1 µM | 0.630 | 0.823 | 0.290 | 0.651 | 2 |

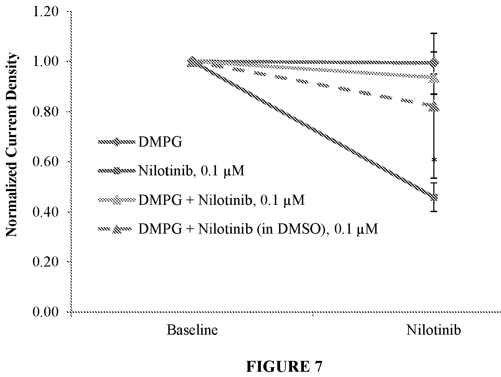

Figure 7 is a graph that shows the effect of DMPG, DMPG + Nilotinib, DMPG + Nilotinib (in DMSO) and Nilotinib on hERG current density from transfected HEK 293 cells.

Active Agent-empty liposomes suspensions. A suspension formulated having a dose of active agent, the empty liposomes (e.g., DMPG, DMPC, or both DMOG and DMPC, and an organoleptic agent, may be formed in suspension and may further includes Xanthan gum (Rhodia Inc.) as the suspending agent and several other ingredients such as, e.g., color, flavor, parabens (e.g., methylparaben and propylparaben) (preservatives), high fructose corn syrup (viscosity builder and sweetener), propylene glycol (solvent and dispersing agent), and ascorbic acid (to adjust the pH of the suspension) were used to achieve a stable suspension. The suspension can be studies for release profiles in 0.1 N HCl at pH 1.2 using USP dissolution apparatus II with 900 ml of dissolution medium. Briefly, samples are withdrawn at predetermined time intervals and were analyzed for active agent content using HPLC analysis. The release of the active agent against time can be plotted.

Different amounts of thixotropic agents (and if necessary salts) can be added to three suspensions to obtain suspensions with varying thixotropic agent, e.g., 0.1, 0.3, and 0.5 weight percent. The suspensions can be mixed and held for 24 hours to achieve equilibrium.

In certain embodiments, the active agents can also be coated and formed into mini-caps, mini-tabs, or just small particles (1.0 micrometer (uM), 10 uM, 100 uM, to 1 millimeter) and mixed in solution with the empty liposomes and the organoleptic and/or thixotropic agent.

It is contemplated that any embodiment discussed in this specification can be implemented with respect to any method, kit, reagent, or composition of the invention, and vice versa. Furthermore, compositions of the invention can be used to achieve methods of the invention.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. In embodiments of any of the compositions and methods provided herein, "comprising" may be replaced with "consisting essentially of" or "consisting of". As used herein, the phrase "consisting essentially of" requires the specified integer(s) or steps as well as those that do not materially affect the character or function of the claimed invention. As used herein, the term "consisting" is used to indicate the presence of the recited integer (e.g., a feature, an element, a characteristic, a property, a method/process step or a limitation) or group of integers (e.g., feature(s), element(s), characteristic(s), propertie(s), method/process steps or limitation(s)) only.

The term "or combinations thereof' as used herein refers to all permutations and combinations of the listed items preceding the term. For example, "A, B, C, or combinations thereof' is intended to include at least one of A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "substantial" or "substantially" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by at least ±1, 2, 3, 4, 5, 6, 7, 10, 12 or 15%.

### REFERENCES

U.S. Patent Publication No. 2010/0004549: System and Method of Serial Comparison for Detection of Long QT Syndrome (LQTS).

U.S. Patent Publication No. 2008/0255464: System and Method for Diagnosing and Treating Long QT Syndrome.

U.S. Patent Publication No. 2007/0048284: Cardiac Arrhythmia Treatment Methods.

U.S. Patent Publication No. 2001/00120890: Ion Channel Modulating Activity I.

## Claims

1. A composition for use in preventing one or more cardiac channelopathies or conditions resulting from irregularities or alterations in cardiac patterns caused by an active agent or a drug in a human or animal subject comprising:
- an amount of a phosphatidylglycerol adapted for oral administration effective to reduce or prevent one or more cardiac channelopathies or conditions resulting from irregularities or alterations in cardiac patterns caused by the active agent or drug; and
- one or more thixotropic agents,
wherein the composition is a liquid and the thixotropic agent forms a thixotrophic matrix and is selected from at least one of polysaccharides, cellulose, carboxymethylcellulose, gums, xanthan gum, collagen, gelatin, aerogels, polyacrylamide, alkyd resins, or silica-lipids;
wherein the active agent or drug is selected from Albuterol (salbutamol), Alfuzosin, Amantadine, Amiodarone, Amisulpride, Amitriptyline, Amoxapine, Amphetamine, Anagrelide, Apomorphine, Arformoterol, Aripiprazole, Arsenic trioxide, Astemizole, Atazanavir, Atomoxetine, Azithromycin, Bedaquiline, Bepridil, Bortezomib, Bosutinib, Chloral hydrate, Chloroquine, Chlorpromazine, Ciprofloxacin, Cisapride, Citalopram, Clarithromycin, Clomipramine, Clozapine, Cocaine, Crizotinib, Dabrafenib, Dasatinib, Desipramine, Dexmedetomidine, Dexmethylphenidate, Dextroamphetamine (d-Amphetamine), Dihydroartemisinin+piperaquine, Diphenhydramine, Disopyramide, Dobutamine, Dofetilide, Dolasetron, Domperidone, Dopamine, Doxepin, Dronedarone, Droperidol, Ephedrine, Epinephrine (Adrenaline), Eribulin, Erythromycin, Escitalopram, Famotidine, Felbamate, Fenfluramine, Fingolimod, Flecainide, Fluconazole, Fluoxetine, Formoterol, Foscarnet, Fosphenytoin, Furosemide (Frusemide), Galantamine, Gatifloxacin, Gemifloxacin, Granisetron, Halofantrine, Haloperidol, Hydrochlorothiazide, Ibutilide, Iloperidone, Imipramine (melipramine), Indapamide, Isoproterenol, Isradipine, Itraconazole, Ivabradine, Ketoconazole, Lapatinib, Levalbuterol (levsalbutamol), Levofloxacin, Levomethadyl, Lisdexamfetamine, Lithium, Mesoridazine, Metaproterenol, Methadone, Methamphetamine (methamfetamine), Methylphenidate, Midodrine, Mifepristone, Mirabegron, Mirtazapine, Moexipril/HCTZ, Moxifloxacin, Nelfinavir, Nicardipine, Nilotinib, Norepinephrine (noradrenaline), Norfloxacin, Nortriptyline, Ofloxacin, Olanzapine, Ondansetron, Oxytocin, Paliperidone, Paroxetine, Pasireotide, Pazopanib, Pentamidine, Perflutren lipid microspheres, Phentermine, Phenylephrine, Phenylpropanolamine, Pimozide, Posaconazole, Probucol, Procainamide, Promethazine, Protriptyline, Pseudoephedrine, Quetiapine, Quinidine, Quinine sulfate, Ranolazine, Rilpivirine, Risperidone, Ritodrine, Ritonavir, Roxithromycin, Salmeterol, Saquinavir, Sertindole, Sertraline, Sevoflurane, Sibutramine, Solifenacin, Sorafenib, Sotalol, Sparfloxacin, Sulpiride, Sunitinib, Tacrolimus, Tamoxifen, Telaprevir, Telavancin, Telithromycin, Terbutaline, Terfenadine, Tetrabenazine, Thioridazine, Tizanidine, Tolterodine, Toremifene, Trazodone, Trimethoprim-Sulfa, Trimipramine, Vandetanib, Vardenafil, Vemurafenib, Venlafaxine, Voriconazole, Vorinostat, or Ziprasidone;
wherein the phosphatidylglycerol is selected from lysophosphatidylcholine, lauroyl-lysophosphatidylcholine, myristoyl-lysophosphatidylcholine, palmitoyl-lysophosphatidylcholine, stearoyl-lysophosphatidylcholine, arachidoyl-lysophosphatidylcholine, oleoyl-lysophosphatidylcholine, linoleoyl-lysophosphatidylcholine, linolenoyl-lysophosphatidylcholine or erucoyl-lysophosphatidylcholine.

2. The composition for use according to claim 1, wherein the composition further comprises one or more organoleptic agents.

3. The composition for use according to claim 1 or 2, wherein the one or more organoleptic agents include one or more flavorants, sweeteners, coolants, dyes, or combinations and mixtures thereof.

4. The composition for use according to any one of claims 1 to 3, wherein the phosphatidylglycerol is formed into empty liposomes and have an average diameter of 10, 20, 25, 30, 40, 50, 60, 75, 80, 90, or 100 nM.

5. The composition for use according to any one of claims 1 to 4, wherein the phosphatidylglycerol include at least one or 1-Myristoyl-2-Hydroxy-sn-Glycero-3-Phosphocholine (DMPC), 12-Mysteroyl-2-Hydroxy-sn-Glycero-3-[Phospho-rac-(glycerol)] (DMPG), DMPC/DMPG, 1-myristoyl-2-hydroxy-*sn-*glycero-3-phospho-(1'-*rac*-glycerol) (LysoPG), or 1-myristoyl-2-hydroxy-*sn*-glycero-3-phosphocholine (LysoPC), wherein the liposomes are optionally formed into liposomes with an average diameter of 10, 20, 25, 30, 40, 50, 60, 75, 80, 90, or 100 nM.

6. The composition for use according to any one of claim 1 to 5, wherein the phosphatidylglycerol is defined further as a short chain fatty acid is up to 5 carbons, a medium chain is 6 to 12 carbons, a long chain is 13-21 carbons and a very long chain fatty acid is greater than 22 carbons, including both even and odd chain fatty acids.

7. The composition for use according to any one of claims 1 to 6, wherein the phosphatidylglycerol has 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55 or more carbons, which are saturated or unsaturated.

8. The composition for use according to any one of claims 1 to 7, wherein the cardiac channelopathy or the condition resulting from the irregularity or alteration in the cardiac pattern is inhibition of an ion channel responsible for the delayed-rectifier K+ current in the heart, polymorphic ventricular tachycardia, prolongation of the QTc, LQT2, LQTS, or torsades de pointes, or is used for the treatment or prevention of prolongation of the IKr channel inhibition or QT prolongation induced by administration of the active agent or drug used in the treatment of cardiac, allergic, or cancer related diseases.

9. A composition for use in preventing or treating one or more cardiac diseases in a human or animal subject caused by an active agent or drug, wherein the active agent or drug are used to treat a disease in a human or animal subject, the composition comprising:
an amount of a lysophosphatidylglycerol adapted for oral administration effective to reduce or prevent the one or more cardiac diseases selected from channelopathies, irregularities or alterations in cardiac patterns, I_{Kr} channel inhibition, or QT prolongation caused by the active agent or drug;
an effective amount of the active agent or drug sufficient to treat the one or more cardiac disease, wherein the orally provided lysophosphatidylglycerol reduces or eliminates the at least one or more cardiac diseases; and
one or more thixotropic agents, wherein the composition is a liquid and the thixotropic agent forms a thixotrophic matrix and is selected from at least one of polysaccharides, cellulose, carboxymethylcellulose, gums, xanthan gum, collagen, gelatin, aerogels, polyacrylamide, alkyd resins, or silica-lipids,
wherein the active agent or drug is selected from Albuterol (salbutamol), Alfuzosin, Amantadine, Amiodarone, Amisulpride, Amitriptyline, Amoxapine, Amphetamine, Anagrelide, Apomorphine, Arformoterol, Aripiprazole, Arsenic trioxide, Astemizole, Atazanavir, Atomoxetine, Azithromycin, Bedaquiline, Bepridil, Bortezomib, Bosutinib, Chloral hydrate, Chloroquine, Chlorpromazine, Ciprofloxacin, Cisapride, Citalopram, Clarithromycin, Clomipramine, Clozapine, Cocaine, Crizotinib, Dabrafenib, Dasatinib, Desipramine, Dexmedetomidine, Dexmethylphenidate, Dextroamphetamine (d-Amphetamine), Dihydroartemisinin+piperaquine, Diphenhydramine, Disopyramide, Dobutamine, Dofetilide, Dolasetron, Domperidone, Dopamine, Doxepin, Dronedarone, Droperidol, Ephedrine, Epinephrine (Adrenaline), Eribulin, Erythromycin, Escitalopram, Famotidine, Felbamate, Fenfluramine, Fingolimod, Flecainide, Fluconazole, Fluoxetine, Formoterol, Foscarnet, Fosphenytoin, Furosemide (Frusemide), Galantamine, Gatifloxacin, Gemifloxacin, Granisetron, Halofantrine, Haloperidol, Hydrochlorothiazide, Ibutilide, Iloperidone, Imipramine (melipramine), Indapamide, Isoproterenol, Isradipine, Itraconazole, Ivabradine, Ketoconazole, Lapatinib, Levalbuterol (levsalbutamol), Levofloxacin, Levomethadyl, Lisdexamfetamine, Lithium, Mesoridazine, Metaproterenol, Methadone, Methamphetamine (methamfetamine), Methylphenidate, Midodrine, Mifepristone, Mirabegron, Mirtazapine, Moexipril/HCTZ, Moxifloxacin, Nelfinavir, Nicardipine, Nilotinib, Norepinephrine (noradrenaline), Norfloxacin, Nortriptyline, Ofloxacin, Olanzapine, Ondansetron, Oxytocin, Paliperidone, Paroxetine, Pasireotide, Pazopanib, Pentamidine, Perflutren lipid microspheres, Phentermine, Phenylephrine, Phenylpropanolamine, Pimozide, Posaconazole, Probucol, Procainamide, Promethazine, Protriptyline, Pseudoephedrine, Quetiapine, Quinidine, Quinine sulfate, Ranolazine, Rilpivirine, Risperidone, Ritodrine, Ritonavir, Roxithromycin, Salmeterol, Saquinavir, Sertindole, Sertraline, Sevoflurane, Sibutramine, Solifenacin, Sorafenib, Sotalol, Sparfloxacin, Sulpiride, Sunitinib, Tacrolimus, Tamoxifen, Telaprevir, Telavancin, Telithromycin, Terbutaline, Terfenadine, Tetrabenazine, Thioridazine, Tizanidine, Tolterodine, Toremifene, Trazodone, Trimethoprim-Sulfa, Trimipramine, Vandetanib, Vardenafil, Vemurafenib, Venlafaxine, Voriconazole, Vorinostat, or Ziprasidone;
wherein the phosphatidylglycerol is selected from lysophosphatidylcholine, lauroyl-lysophosphatidylcholine, myristoyl-lysophosphatidylcholine, palmitoyl-lysophosphatidylcholine, stearoyl-lysophosphatidylcholine, arachidoyl-lysophosphatidylcholine, oleoyl-lysophosphatidylcholine, linoleoyl-lysophosphatidylcholine, linolenoyl-lysophosphatidylcholine or erucoyl-lysophosphatidylcholine.

## Patentansprüche

1. Zusammensetzung zur Anwendung bei der Vorbeugung einer oder mehrerer kardialer Kanalopathien oder Zustände, die aus Unregelmäßigkeiten oder Änderungen in kardialen Mustern resultieren, die durch einen Wirkstoff oder ein Arzneimittel verursacht werden, bei einem menschlichen oder tierischen Individuum, aufweisend:
- eine Menge eines Phosphatidylglycerols, das zur oralen Verabreichung angepasst und zum Verringern oder Vorbeugen einer oder mehrerer kardialer Kanalopathien oder Zustände wirksam ist, die aus Unregelmäßigkeiten oder Änderungen in kardialen Mustern resultieren, die durch den Wirkstoff oder das Arzneimittel verursacht werden, und
- ein oder mehrere thixotrope Mittel,
wobei die Zusammensetzung eine Flüssigkeit ist und das thixotropes Mittel eine thixotrope Matrix bildet und aus mindestens einem von Polysacchariden, Cellulose, Carboxymethylcellulose, Gummis, Xanthangummi, Kollagen, Gelatine, Aerogelen, Polyacrylamid, Alkydharzen oder Siliciumdioxid-Lipiden ausgewählt ist;
wobei der Wirkstoff oder das Arzneimittel aus Albuterol (Salbutamol), Alfuzosin, Amantadin, Amiodaron, Amisulprid, Amitriptylin, Amoxapin, Amphetamin, Anagrelid, Apomorphin, Arformoterol, Aripiprazol, Arsentrioxid, Astemizol, Atazanavir, Atomoxetin, Azithromycin, Bedaquilin, Bepridil, Bortezomib, Bosutinib, Chlorhydrat, Chloroquin, Chlorpromazin, Ciprofloxacin, Cisaprid, Citalopram, Clarithromycin, Clomipramin, Clozapin, Kokain, Crizotinib, Dabrafenib, Dasatinib, Desipramin, Dexmedetomidin, Dexmethylphenidat, Dextroamphetamin (d-Amphetamin), Dihydroartemisinin und Piperaquin, Diphenhydramin, Disopyramid, Dobutamin, Dofetilid, Dolasetron, Domperidon, Dopamin, Doxepin, Dronedaron, Droperidol, Ephedrin, Epinephrin (Adrenalin), Eribulin, Erythromycin, Escitalopram, Famotidin, Felbamat, Fenfluramin, Fingolimod, Flecainid, Fluconazol, Fluoxetin, Formoterol, Foscarnet, Fosphenytoin, Furosemid (Frusemid), Galantamin, Gatifloxacin, Gemifloxacin, Granisetron, Halofantrin, Haloperidol, Hydrochlorothiazid, Ibutilid, Iloperidon, Imipramin (Melipramin), Indapamid, Isoproterenol, Isradipin, Itraconazol, Ivabradin, Ketoconazol, Lapatinib, Levalbuterol (Levsalbutamol), Levofloxacin, Levomethadyl, Lisdexamfetamin, Lithium, Mesoridazin, Metaproterenol, Methadon, Methamphetamin (Methamfetamin), Methylphenidat, Midodrin, Mifepriston, Mirabegron, Mirtazapin, Moexipril/HCTZ, Moxifloxacin, Nelfinavir, Nicardipin, Nilotinib, Norepinephrin (Noradrenalin), Norfloxacin, Nortriptylin, Ofloxacin, Olanzapin, Ondansetron, Oxytocin, Paliperidon, Paroxetin, Pasireotid, Pazopanib, Pentamidin, Perflutren-Lipid-Mikrokügelchen, Phentermin, Phenylephrin, Phenylpropanolamin, Pimozid, Posaconazol, Probucol, Procainamid, Promethazin, Protriptylin, Pseudoephedrin, Quetiapin, Chinidin, Chininsulfat, Ranolazin, Rilpivirin, Risperidon, Ritodrin, Ritonavir, Roxithromycin, Salmeterol, Saquinavir, Sertindol, Sertralin, Sevofluran, Sibutramin, Solifenacin, Sorafenib, Sotalol, Sparfloxacin, Sulpirid, Sunitinib, Tacrolimus, Tamoxifen, Telaprevir, Telavancin, Telithromycin, Terbutalin, Terfenadin, Tetrabenazin, Thioridazin, Tizanidin, Tolterodin, Toremifen, Trazodon, Trimethoprim-Sulfa, Trimipramin, Vandetanib, Vardenafil, Vemurafenib, Venlafaxin, Voriconazol, Vorinostat oder Ziprasidon ausgewählt ist;
wobei das Phosphatidylglycerol aus Lysophosphatidylcholin, Lauroyl-Lysophosphatidylcholin, Myristoyl-Lysophosphatidylcholin, Palmitoyl-Lysophosphatidylcholin, Stearoyl-Lysophosphatidylcholin, Arachidoyl-Lysophosphatidylcholin, Oleoyl-Lysophosphatidylcholin, Linoleoyl-Lysophosphatidylcholin, Linolenoyl-Lysophosphatidylcholin oder Erucoyl-Lysophosphatidylcholin ausgewählt ist.

2. Zusammensetzung zur Anwendung nach Anspruch 1, wobei die Zusammensetzung ferner ein oder mehrere organoleptische Mittel aufweist.

3. Zusammensetzung zur Anwendung nach Anspruch 1 oder 2, wobei das eine oder die mehreren organoleptischen Mittel einen oder mehrere Geschmacksstoffe, Süßstoffe, Kühlmittel, Farbstoffe oder Kombinationen und Gemische davon einschließen.

4. Zusammensetzung zur Anwendung nach einem der Ansprüche 1 bis 3, wobei das Phosphatidylglycerol zu leeren Liposomen ausgebildet ist und einen durchschnittlichen Durchmesser von 10, 20, 25, 30, 40, 50, 60, 75, 80, 90 oder 100 nM hat.

5. Zusammensetzung zur Anwendung nach einem der Ansprüche 1 bis 4, wobei das Phosphatidylglycerol mindestens eines von 1-Myristoyl-2-hydroxy-sn-glycero-3-phosphocholin (DMPC), 12-Myristoyl-2-hydroxy-sn-glycero-3-[phospho-rac-(glycerol)] (DMPG), DMPC/DMPG, 1-Myristoyl-2-hydroxy-*sn*-glycero-3-phospho-(1'-*rac-*glycerol) (LysoPG) oder 1-Myristoyl-2-hydroxy-sn-glycero-3-phosphocholin (LysoPC) einschließt, wobei die Liposomen gegebenenfalls zu Liposomen mit einem durchschnittlichen Durchmesser von 10, 20, 25, 30, 40, 50, 60, 75, 80, 90 oder 100 nM ausgebildet sind.

6. Zusammensetzung zur Anwendung nach einem der Ansprüche 1 bis 5, wobei das Phosphatidylglycerol ferner als eine kurzkettige Fettsäure mit bis zu 5 Kohlenstoffatomen, eine mittelkettige mit 6 bis 12 Kohlenstoffatomen, eine langkettige mit 13 bis 21 Kohlenstoffatomen und eine sehr langkettige Fettsäure mit mehr als 22 Kohlenstoffatomen definiert ist, wobei sowohl geradkettige als auch ungeradkettige Fettsäuren eingeschlossen sind.

7. Zusammensetzung zur Anwendung nach einem der Ansprüche 1 bis 6, wobei das Phosphatidylglycerol 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55 oder mehr Kohlenstoffatome hat, die gesättigt oder ungesättigt sind.

8. Zusammensetzung zur Anwendung nach einem der Ansprüche 1 bis 7, wobei die kardiale Kanalopathie oder der Zustand, der aus der Unregelmäßigkeit oder Veränderung im kardialen Muster resultiert, die Hemmung eines Ionenkanals ist, der für den verzögerten Gleichrichter-K+-Strom im Herzen, polymorphe ventrikuläre Tachykardie, Verlängerung der QTc, LQT2, LQTS oder Torsades de Pointes verantwortlich ist, oder zur Anwendung bei der Behandlung oder Vorbeugung der Verlängerung der Hemmung des IKr-Kanals oder QT-Verlängerung, die durch Verabreichung des Wirkstoffs oder des Arzneimittels, das bei der Behandlung von kardialen, allergischen oder durch Krebs bedingten Krankheiten angewendet wird, ausgelöst wird.

9. Zusammensetzung zur Anwendung bei der Vorbeugung oder Behandlung einer oder mehrerer Herzerkrankungen bei einem menschlichen oder tierischen Individuum, die durch einen Wirkstoff oder ein Arzneimittel verursacht werden, wobei der Wirkstoff oder das Arzneimittel zum Behandeln einer Erkrankung bei einem menschlichen oder tierischen Individuum angewendet wird, wobei die Zusammensetzung Folgendes aufweist:
eine Menge eines Lysophosphatidylglycerols, die zur oralen Verabreichung angepasst und zum Verringern oder Vorbeugen einer oder mehrerer Herzerkrankungen ausgewählt aus Kanalopathien, Unregelmäßigkeiten oder Änderungen in kardialen Mustern, Hemmung des I_{Kr}-Kanals oder QT-Verlängerung, die durch den Wirkstoff oder das Arzneimittel verursacht werden, wirksam ist;
eine wirksame Menge des Wirkstoffs oder Arzneimittels, die ausreicht, um die eine oder mehreren Herzerkrankungen zu behandeln, wobei das oral bereitgestellte Lysophosphatidylglycerol die mindestens eine oder mehreren Herzerkrankungen verringert oder beseitigt; und
ein oder mehrere thixotrope Mittel, wobei die Zusammensetzung eine Flüssigkeit ist und das thixotropes Mittel eine thixotrope Matrix bildet und aus mindestens einem von Polysacchariden, Cellulose, Carboxymethylcellulose, Gummis, Xanthangummi, Kollagen, Gelatine, Aerogelen, Polyacrylamid, Alkydharzen oder Siliciumdioxid-Lipiden ausgewählt ist,
wobei der Wirkstoff oder das Arzneimittel aus Albuterol (Salbutamol), Alfuzosin, Amantadin, Amiodaron, Amisulprid, Amitriptylin, Amoxapin, Amphetamin, Anagrelid, Apomorphin, Arformoterol, Aripiprazol, Arsentrioxid, Astemizol, Atazanavir, Atomoxetin, Azithromycin, Bedaquilin, Bepridil, Bortezomib, Bosutinib, Chlorhydrat, Chloroquin, Chlorpromazin, Ciprofloxacin, Cisaprid, Citalopram, Clarithromycin, Clomipramin, Clozapin, Kokain, Crizotinib, Dabrafenib, Dasatinib, Desipramin, Dexmedetomidin, Dexmethylphenidat, Dextroamphetamin (d-Amphetamin), Dihydroartemisinin und Piperaquin, Diphenhydramin, Disopyramid, Dobutamin, Dofetilid, Dolasetron, Domperidon, Dopamin, Doxepin, Dronedaron, Droperidol, Ephedrin, Epinephrin (Adrenalin), Eribulin, Erythromycin, Escitalopram, Famotidin, Felbamat, Fenfluramin, Fingolimod, Flecainid, Fluconazol, Fluoxetin, Formoterol, Foscarnet, Fosphenytoin, Furosemid (Frusemid), Galantamin, Gatifloxacin, Gemifloxacin, Granisetron, Halofantrin, Haloperidol, Hydrochlorothiazid, Ibutilid, Iloperidon, Imipramin (Melipramin), Indapamid, Isoproterenol, Isradipin, Itraconazol, Ivabradin, Ketoconazol, Lapatinib, Levalbuterol (Levsalbutamol), Levofloxacin, Levomethadyl, Lisdexamfetamin, Lithium, Mesoridazin, Metaproterenol, Methadon, Methamphetamin (Methamfetamin), Methylphenidat, Midodrin, Mifepriston, Mirabegron, Mirtazapin, Moexipril/HCTZ, Moxifloxacin, Nelfinavir, Nicardipin, Nilotinib, Norepinephrin (Noradrenalin), Norfloxacin, Nortriptylin, Ofloxacin, Olanzapin, Ondansetron, Oxytocin, Paliperidon, Paroxetin, Pasireotid, Pazopanib, Pentamidin, Perflutren-Lipid-Mikrokügelchen, Phentermin, Phenylephrin, Phenylpropanolamin, Pimozid, Posaconazol, Probucol, Procainamid, Promethazin, Protriptylin, Pseudoephedrin, Quetiapin, Chinidin, Chininsulfat, Ranolazin, Rilpivirin, Risperidon, Ritodrin, Ritonavir, Roxithromycin, Salmeterol, Saquinavir, Sertindol, Sertralin, Sevofluran, Sibutramin, Solifenacin, Sorafenib, Sotalol, Sparfloxacin, Sulpirid, Sunitinib, Tacrolimus, Tamoxifen, Telaprevir, Telavancin, Telithromycin, Terbutalin, Terfenadin, Tetrabenazin, Thioridazin, Tizanidin, Tolterodin, Toremifen, Trazodon, Trimethoprim-Sulfa, Trimipramin, Vandetanib, Vardenafil, Vemurafenib, Venlafaxin, Voriconazol, Vorinostat oder Ziprasidon ausgewählt ist;
wobei das Phosphatidylglycerol aus Lysophosphatidylcholin, Lauroyl-Lysophosphatidylcholin, Myristoyl-Lysophosphatidylcholin, Palmitoyl-Lysophosphatidylcholin, Stearoyl-Lysophosphatidylcholin, Arachidoyl-Lysophosphatidylcholin, Oleoyl-Lysophosphatidylcholin, Linoleoyl-Lysophosphatidylcholin, Linolenoyl-Lysophosphatidylcholin oder Erucoyl-Lysophosphatidylcholin ausgewählt ist.

## Revendications

1. Composition destinée à être utilisée dans la prévention d'une ou plusieurs canalopathies cardiaques ou affections résultant d'irrégularités ou d'altérations dans les schémas cardiaques causées par un agent actif ou un médicament chez un sujet humain ou animal, comprenant:
- une quantité d'un phosphatidylglycérol adaptée pour une administration orale efficace pour réduire ou prévenir une ou plusieurs canalopathies cardiaques ou affections résultant d'irrégularités ou d'altérations dans les schémas cardiaques causées par l'agent actif ou le médicament; et
- un ou plusieurs agents thixotropes,
où la composition est un liquide et l'agent thixotrope forme une matrice thixotrope et est choisi parmi au moins l'un parmi les polysaccharides, la cellulose, la carboxyméthylcellulose, les gommes, la gomme xanthane, le collagène, la gélatine, les aérogels, le polyacrylamide, les résines alkydes ou les silice-lipides;
où l'agent actif ou le médicament est choisi parmi l'albutérol (salbutamol), l'alfuzosine, l'amantadine, l'amiodarone, l'amisulpride, l'amitriptyline, l'amoxapine, l'amphétamine, l'anagrélide, l'apomorphine, l'arformotérol, l'aripiprazole, le trioxyde d'arsenic, l'astémizole, l'atazanavir, l'atomoxétine, l'azithromycine, la bédaquiline, le bépridil, le bortézomib, le bosutinib, l'hydrate de chloral, la chloroquine, la chlorpromazine, la ciprofloxacine, le cisapride, le citalopram, la clarithromycine, la clomipramine, la clozapine, la cocaïne, le crizotinib, le dabrafenib, le dasatinib, la désipramine, la dexmédétomidine, le dexméthylphénidate, la dextroamphétamine (d-amphétamine), la dihydroartémisinine + pipéraquine, la diphenhydramine, le disopyramide, la dobutamine, le dofétilide, le dolasétron, la dompéridone, la dopamine, la doxépine, la dronédarone, le dropéridol, l'éphédrine, l'épinéphrine (adrénaline), l'éribuline, l'érythromycine, l'escitalopram, la famotidine, le felbamate, la fenfluramine, le fingolimod, la flécaïnide, le fluconazole, la fluoxétine, le formotérol, le foscarnet, la fosphénytôine, le furosémide (frusémide), la galantamine, la gatifloxacine, la gémifloxacine, le granisétron, l'halofantrine, l'halopéridol, l'hydrochlorothiazide, l'ibutilide, l'ilopéridone, l'imipramine (mélipramine), l'indapamide, l'isoprotérénol, l'isradipine, l'itraconazole, l'ivabradine, le kétoconazole, le lapatinib, le lévalbutérol (lévsalbutamol), la lévofloxacine, le lévométhadyl, la lisdexamfétamine, le lithium, la mésoridazine, le métaprotérénol, la méthadone, la méthamphétamine (méthamfétamine), le méthylphénidate, la midodrine, la mifépristone, le mirabegron, la mirtazapine, le moexipril/HCTZ, la moxifloxacine, le nelfinavir, la nicardipine, le nilotinib, la norépinéphrine (noradrénaline), la norfloxacine, la nortriptyline, l'ofloxacine, l'olanzapine, l'ondansétron, l'ocytocine, la palipéridone, la paroxétine, le pasiréotide, le pazopanib, la pentamidine, les microsphères lipidiques de perflutren, la phentermine, la phényléphrine, la phénylpropanolamine, le pimozide, le posaconazole, le probucol, le procainamide, la prométhazine, la protriptyline, la pseudoéphédrine, la quétiapine, la quinidine, le sulfate de quinine, la ranolazine, la rilpivirine, la rispéridone, la ritodrine, le ritonavir, la roxithromycine, le salmétérol, le saquinavir, le sertindole, la sertraline, le sévoflurane, la sibutramine, la solifénacine, le sorafénib, le sotalol, la sparfloxacine, le sulpiride, le sunitinib, le tacrolimus, le tamoxifène, le telaprevir, la telavancine, la télithromycine, la terbutaline, la terfénadine, la tétrabénazine, la thioridazine, la tizanidine, la toltérodine, le torémifène, la trazodone, le triméthoprime-sulfa, la trimipramine, le vandétanib, le vardénafil, le vémurafénib, la venlafaxine, le voriconazole, le vorinostat ou la ziprasidone;
où le phosphatidylglycérol est choisi parmi la lysophosphatidylcholine, la lauroyl-lysophosphatidylcholine, la myristoyl-lysophosphatidylcholine, la palmitoyl-lysophosphatidylcholine, la stéaroyl-lysophosphatidylcholine, l'arachidoyl-lysophosphatidylcholine, l'oléoyl-lysophosphatidylcholine, la linoléoyl-lysophosphatidylcholine, la linolénoyl-lysophosphatidylcholine ou l'érucoyl-lysophosphatidylcholine.

2. Composition destinée à être utilisée selon la revendication 1, où la composition comprend en outre un ou plusieurs agents organoleptiques.

3. Composition destinée à être utilisée selon la revendication 1 ou 2, où les un ou plusieurs agents organoleptiques incluent un ou plusieurs aromatisants, édulcorants, réfrigérants, colorants ou leurs combinaisons et mélanges.

4. Composition destinée à être utilisée selon l'une quelconque des revendications 1 à 3, où le phosphatidylglycérol est formé en liposomes vides et ont un diamètre moyen de 10, 20, 25, 30, 40, 50, 60, 75, 80, 90 ou 100 nM.

5. Composition destinée à être utilisée selon l'une quelconque des revendications 1 à 4, où le phosphatidylglycérol inclut au moins un ou la 1-myristoyl-2-hydroxy-sn-glycéro-3-phosphocholine (DMPC), le 12-mystéroyl-2-hydroxy-sn-glycéro-3-[phospho-rac-(glycérol)] (DMPG), DMPC/DMPG, le 1-myristoyl-2-hydroxy-*sn*-glycéro-3-phospho-(1'-*rac-*glycérol) (LysoPG), ou la 1-myristoyl-2-hydro-*sn*-sirglycéro-3-phosphocholine (LysoPC), où les liposomes sont éventuellement formés en liposomes d'un diamètre moyen de 10 20, 25, 30, 40, 50, 60, 75, 80, 90 ou 100 nM.

6. Composition destinée à être utilisée selon l'une quelconque des revendications 1 à 5, où le phosphatidylglycérol est défini en outre comme un acide gras à chaîne courte est jusqu'à 5 carbones, une chaîne moyenne est de 6 à 12 carbones, une chaîne longue est de 13-21 carbones et un acide gras à très longue chaîne est supérieur à 22 carbones, y compris les acides gras à chaîne paire et impaire.

7. Composition destinée à être utilisée selon l'une quelconque des revendications 1 à 6, où le phosphatidylglycérol a 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55 carbones ou plus, qui sont saturés ou insaturés.

8. Composition destinée à être utilisée selon l'une quelconque des revendications 1 à 7, où la canalopathie cardiaque ou l'affection résultant de l'irrégularité ou de l'altération dans le schéma cardiaque est l'inhibition d'un canal ionique responsable du courant K⁺ redresseur retardé dans le coeur, la tachycardie ventriculaire polymorphe, la prolongation du QTc, LOT2, LOTS ou les torsades de pointes; ou est utilisée pour le traitement ou la prévention de la prolongation de l'inhibition du canal IKr ou la prolongation de QT induite par l'administration de l'agent actif ou du médicament utilisé dans le traitement des maladies cardiaques, allergiques ou liées au cancer.

9. Composition destinée à être utilisée pour prévenir ou traiter une ou plusieurs maladies cardiaques chez un sujet humain ou animal causées par un agent actif ou un médicament, où l'agent actif ou le médicament est utilisé pour traiter une maladie chez un sujet humain ou animal, la composition comprenant:
une quantité d'un lysophosphatidylglycérol adaptée pour une administration orale efficace pour réduire ou prévenir les une ou plusieurs maladies cardiaques choisies parmi les canalopathies, les irrégularités ou les altérations des schémas cardiaques, l'inhibition du canal I_{Kr} ou la prolongation de QT causée par l'agent actif ou le médicament;
une quantité efficace de l'agent actif ou du médicament suffisante pour traiter les une ou plusieurs maladies cardiaques, où le lysophosphatidylglycérol administré par voie orale réduit ou élimine les au moins une ou plusieurs maladies cardiaques; et
un ou plusieurs agents thixotropes, où la composition est un liquide et l'agent thixotrope forme une matrice thixotrope et est choisi parmi au moins l'un parmi les polysaccharides, la cellulose, la carboxyméthylcellulose, les gommes, la gomme xanthane, le collagène, la gélatine, les aérogels, le polyacrylamide, les résines alkydes, ou les silice-lipides,
où l'agent actif ou le médicament est choisi parmi l'albutérol (salbutamol), l'alfuzosine, l'amantadine, l'amiodarone, l'amisulpride, l'amitriptyline, l'amoxapine, l'amphétamine, l'anagrélide, l'apomorphine, l'arformotérol, l'aripiprazole, le trioxyde d'arsenic, l'astémizole, l'atazanavir, l'atomoxétine, l'azithromycine, la bédaquiline, le bépridil, le bortézomib, le bosutinib, l'hydrate de chloral, la chloroquine, la chlorpromazine, la ciprofloxacine, le cisapride, le citalopram, la clarithromycine, la clomipramine, la clozapine, la cocaïne, le crizotinib, le dabrafenib, le dasatinib, la désipramine, la dexmédétomidine, le dexméthylphénidate, la dextroamphétamine (d-amphétamine), la dihydroartémisinine + pipéraquine, la diphenhydramine, le disopyramide, la dobutamine, le dofétilide, le dolasétron, la dompéridone, la dopamine, la doxépine, la dronédarone, le dropéridol, l'éphédrine, l'épinéphrine (adrénaline), l'éribuline, l'érythromycine, l'escitalopram, la famotidine, le felbamate, la fenfluramine, le fingolimod, la flécaïnide, le fluconazole, la fluoxétine, le formotérol, le foscarnet, la fosphénytoïne, le furosémide (frusémide), la galantamine, la gatifloxacine, la gémifloxacine, le granisétron, l'halofantrine, l'halopéridol, l'hydrochlorothiazide, l'ibutilide, l'ilopéridone, l'imipramine (mélipramine), l'indapamide, l'isoprotérénol, l'isradipine, l'itraconazole, l'ivabradine, le kétoconazole, le lapatinib, le lévalbutérol (lévsalbutamol), la lévofloxacine, le lévométhadyl, la lisdexamfétamine, le lithium, la mésoridazine, le métaprotérénol, la méthadone, la méthamphétamine (méthamfétamine), le méthylphénidate, la midodrine, la mifépristone, le mirabegron, la mirtazapine, le moexipril/HCTZ, la moxifloxacine, le nelfinavir, la nicardipine, le nilotinib, la norépinéphrine (noradrénaline), la norfloxacine, la nortriptyline, l'ofloxacine, l'olanzapine, l'ondansétron, l'ocytocine, la palipéridone, la paroxétine, le pasiréotide, le pazopanib, la pentamidine, les microsphères lipidiques de perflutren, la phentermine, la phényléphrine, la phénylpropanolamine, le pimozide, le posaconazole, le probucol, le procaïnamide, la prométhazine, la protriptyline, la pseudoéphédrine, la quétiapine, la quinidine, le sulfate de quinine, la ranolazine, la rilpivirine, la rispéridone, la ritodrine, le ritonavir, la roxithromycine, le salmétérol, le saquinavir, le sertindole, la sertraline, le sévoflurane, la sibutramine, la solifénacine, le sorafénib, le sotalol, la sparfloxacine, le sulpiride, le sunitinib, le tacrolimus, le tamoxifène, le telaprevir, la telavancine, la télithromycine, la terbutaline, la terfénadine, la tétrabénazine, la thioridazine, la tizanidine, la toltérodine, le torémifène, la trazodone, le triméthoprime-sulfa, la trimipramine, le vandétanib, le vardénafil, le vémurafénib, la venlafaxine, le voriconazole, le vorinostat ou la ziprasidone,
où le phosphatidylglycérol est choisi parmi la lysophosphatidylcholine, la lauroyl-lysophosphatidylcholine, la myristoyl-lysophosphatidylcholine, la palmitoyl-lysophosphatidylcholine, la stéaroyl-lysophosphatidylcholine, l'arachidoyl-lysophosphatidylcholine, l'oléoyl-lysophosphatidylcholine, la linoléoyl-lysophosphatidylcholine, la linolénoyl-lysophosphatidylcholine ou l'érucoyl-lysophosphatidylcholine.
